(19) Europäisches Patentamt
European Patent Office
Office européen des brevets

(11) Veröffentlichungsnummer: **0 251 994**
**A1**

(12) # EUROPÄISCHE PATENTANMELDUNG

(21) Anmeldenummer: **87810297.9**

(22) Anmeldetag: **11.05.87**

(51) Int.Cl.[3]: **C 07 D 213/53**
C 07 D 213/38, C 07 D 319/06
C 07 D 405/12, C 07 C 87/28
C 08 F 26/00, B 01 J 31/00

(30) Priorität: **16.05.86 CH 1985/86**

(43) Veröffentlichungstag der Anmeldung:
**07.01.88 Patentblatt 88/1**

(84) Benannte Vertragsstaaten:
**AT BE CH DE FR GB IT LI NL SE**

(71) Anmelder: **CIBA-GEIGY AG**
**Klybeckstrasse 141**
**CH-4002 Basel(CH)**

(72) Erfinder: **Kaschig, Jürgen, Dr.**
**Rötebuckweg 30**
**7800 Freiburg(DE)**

(54) **Optisch aktive Styrolderivate, Polymere daraus, Komplexe mit Iridium (I) und deren Verwendung.**

(57) Optisch aktive Verbindungen der Formel I

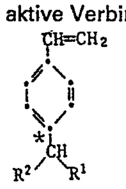

worin $R^1$ $C_1$-$C_4$-Alkyl, Phenyl oder Benzyl bedeutet, $R^2$ einen Rest der Formeln II oder IIa

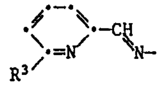 (II), 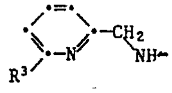 (IIa),

darstellt, worin $R^3$ für H oder $-CH_3$ steht, oder $R^1$ und $R^2$ zusammen ein Rest der Formel

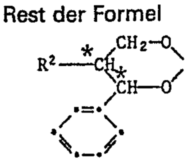

sind, worin $R^2$ die zuvor angegebene Bedeutung hat; und * für überwiegend R- oder überwiegend S-Konfiguration steht.

Die Verbindungen können zu Homo- oder Copolymeren polymerisiert werden. Die Verbindungen und die Polymeren können mit Iridium (I)-Salzen in Gegenwart eines Diens komplexiert werden. Die Komplexe eignen sich als enantioselektive Katalysatoren.

EP 0 251 994 A1

CIBA-GEIGY AG
Basel (Schweiz)

6-15879/=

# Optisch aktive Styrolderivate, Polymere daraus, Komplexe mit Iridium (I) und deren Verwendung

Die Erfindung betrifft optisch aktive Styrolderivate mit einer 2-Pyridinaldiminogruppe oder 2-(Aminomethyl)pyridingruppe, deren Homo- und Copolymere mit olefinischen Comonomeren, Komplexe der Monomeren und Polymeren mit Iridium (I), sowie deren Verwendung als enantioselektive Katalysatoren für die Transferhydrierung von prochiralen Ketonen mit sekundären Alkoholen.

P. Hodge et al. beschreiben in Polymer-supported Reactions in Organic Synthesis, John Wiley & Sons (1980), S. 281 - 283, an Polymere gebundene Rhodiumkomplexe von 2,3-O-Isopropyliden-2,3-dihydroxy-1,4-bis(diphenylphosphinobutan) (DIOP) als enantioselektive Katalysatoren für Hydrierungen.

K. Ohkubo et al. beschreiben in Inorg. Nucl. Chem. Letters, Vol 17, S. 215 - 218 (1981) an Amberlit gebundene Rhodiumkomplexe von DIOP, die als enantioselektive Hydrierkatalysatoren für prochirale Ketone geeignet sind.

G. Zassinovich et al. beschreiben im Journal of Organometallic Chemistry, 222, S. 323 - 329 (1981) kationische Iridium(I)-Komplexe mit einem 1,5-Cyclooctadienliganden und einem 2-Pyridinaldiminliganden, der am Imin-N-Atom durch optisch aktives α-Phenylethyl oder Pinan-3-methyl substituiert ist. Sie wirken als enantioselektive

homogene Katalysatoren bei der Transferhydrierung von prochiralen Ketonen mit Isopropanol. Bei der Reaktion werden zwar hohe Ausbeuten erzielt, aber die optische Ausbeute (Enantiomerenüberschuss) ist relativ gering.

Ein Gegenstand der Erfindung sind optisch aktive Verbindungen der Formel I

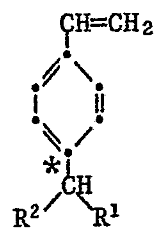 (I),

worin $R^1$ $C_1$-$C_4$-Alkyl, Phenyl oder Benzyl bedeutet, $R^2$ einen Rest der Formeln II oder IIa

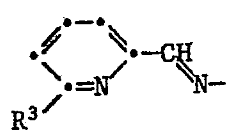 (II), 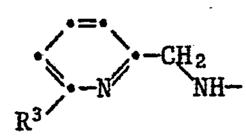 (IIa),

darstellt, worin $R^3$ für H oder $-CH_3$ steht, oder $R^1$ und $R^2$ zusammen ein Rest der Formel

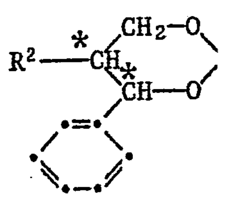

sind, worin $R^2$ die zuvor angegebene Bedeutung hat; und * für überwiegend R- oder überwiegend S-Konfiguration steht.

Bei $R^1$ als $C_1$-$C_4$-Alkyl kann es sich um verzweigtes, bevorzugt lineares Alkyl handeln. Beispiele sind Methyl, Ethyl, n-Propyl, i-Propyl, n-, i- und t-Butyl. Bevorzugt ist $R^1$ Methyl oder Benzyl.

Die Verbindungen der Formel I sind z.B. erhältlich, indem man zur Herstellung der Verbindungen der Formel I, worin $R^2$ ein Rest der Formel II ist, einen Pyridinaldehyd der Formel III

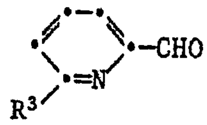

(III)

mit einem Amin der Formel IV

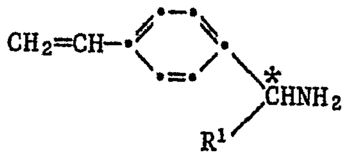

(IV)

oder mit einem Amin der Formel V

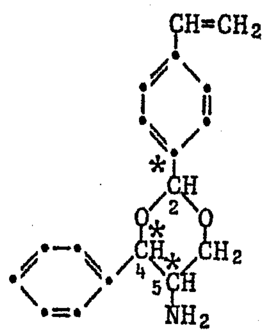

(V)

umsetzt, und zur Herstellung von Verbindungen der Formel I, worin $R^2$ ein Rest der Formel IIa ist, die gebildeten Pyridinaldimine mit $LiAlH_4$ hydriert, wobei $R^1$ und $R^3$ die zuvor angegebene Bedeutung haben.

Die Umsetzung wird zweckmässig in einem Lösungmittel und vorzugsweise bei erhöhter Temperatur, insbesondere bei 40 - 120°C, durchgeführt. Geeignete Lösungmittel sind z.B. Kohlenwasserstoffe (z.B. Pentan, Hexan, Cyclohexan, Methylcyclohexan, Benzol, Toluol und Xylol), Halogenkohlenwasserstoffe (z.B. Chloroform, Methylenchlorid, Tetrachlorkohlenstoff, 1,1,2,2-Tetrachlorethan), Ether (z.B. Diethylether, Dioxan, Tetrahydrofuran) und Alkohole (z.B. Methanol, Ethanol und n-Propanol). Vorteilhaft werden solche Lösungsmittel eingesetzt, mit denen das entstehende Reaktionswasser durch azeotrope Destillation aus dem Reaktionsmedium entfernt werden kann. Die Isolierung der gewünschten Verbindungen erfolgt zweckmässig durch Destillation bzw. Kristallisation. Die Umsetzung der gebildeten Imine der Formel I, worin $R^2$ ein Rest der Formel II ist,

mit $LiAlH_4$ zur Herstellung der Amine der Formel I, worin $R^2$ ein Rest der Formel IIa ist, wird zweckmässig in an sich bekannter Weise bei einer Temperatur von vorzugsweise -20 bis 30°C in einem Ether (z.B. Diethylether, Dioxan oder Tetrahydrofuran) durchgeführt. Zur Isolierung der gewünschten Verbindungen wird das Reaktionsgemisch hydrolysiert, die organische Phase abgetrennt und anschliessend chromatographiert oder destilliert.

Die Pyridinaldehyde der Formel III sind bekannt und käuflich.

Die neuen Verbindungen der Formel IV, worin $R^1$ $C_1$-$C_4$-Alkyl, Phenyl oder Benzyl ist, sind nach dem in G. Wulff et al., Makromol. Chem. 183, S. 2459 - 2467 (1982) beschriebenen Verfahren erhältlich. Es wurde gefunden, dass die optische Drehung der dort angegebenen (R)- bzw. (S)-1-(4-Vinylphenyl)-ethylamine zu niedrig ist und demzufolge Gemische mit einem Ueberschuss je eines stereoisomeren Amins offenbart sind. Die Werte der optischen Drehung für die reinen Stereoisomeren liegen jeweils etwa 12° höher. Die für das von G. Wulff et al. beschriebene Verfahren benötigten Ausgangsprodukte der Formel

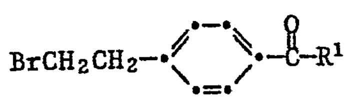

werden nach dem Verfahren von E.L. Foreman in JACS 62, S. 1436 (1940) erhalten. Die klassische Trennmethode der racemischen Reaktionsprodukte als Salze der reinen Stereoisomeren optisch aktiver organischer Säuren durch fraktionierte Kristallisation führt zu den optisch reinen Aminen mit R- bzw. S-Konfiguration.

Das neue Amin der Formel V wird analog zu der von K. Weinges et al. in Chem.Ber. 113, S. 710 - 721 (1980) beschriebenen Verfahren durch Umsetzung von (+)-2-Amino-1-phenyl-1,3-propandiol-hydrobromid mit p-Styrylaldehyd erhalten.

Ein weiterer Gegenstand der Erfindung sind reine stereoisomere optisch aktive Amine der Formeln IV oder V

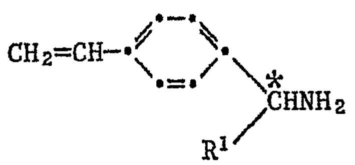 (IV),

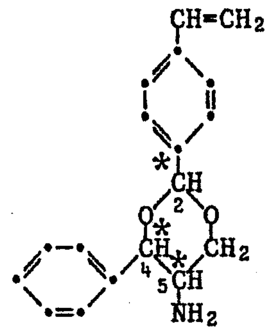 (V)

worin $R^1$ $C_1$-$C_4$-Alkyl, Phenyl oder Benzyl bedeutet und das chirale *C-Atom in Formel IV entweder R- oder S-Konfiguration und die chiralen C-Atome in Formel V 2R,4S,5S-Konfiguration aufweisen. Die Verbindungen der Formel I eignen sich als N,N-Chelatliganden zur Herstellung von Komplexen.

Ein weiterer Gegenstand der Erfindung sind Iridiumkomplexe mit Verbindungen der Formel I, dadurch gekennzeichnet, dass der Komplex den Formeln VI oder VIa entspricht,

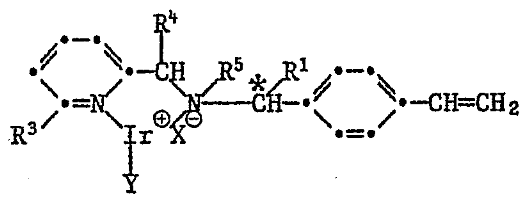 (VI),

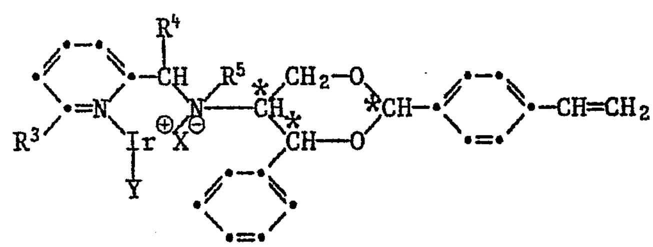 (VIa),

worin * für überwiegend R- oder überwiegend S-Konfiguration steht, $R^1$ und $R^3$ die zuvor angegebene Bedeutung haben, $R^4$ und $R^5$ je für H stehen oder zusammen eine Bindung darstellen, $X^{\ominus}$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten. Die Verbindungen der Formeln VI und VIa können auch in hydratisierter Form vorliegen.

Bevorzugt ist $R^1$ in Formel VI Methyl oder Benzyl.

Bei $X^{\ominus}$ als Anion einer einbasischen anorganischen oder organischen Säure kann es sich zum Beispiel um $F^{\ominus}$, $Cl^{\ominus}$, $Br^{\ominus}$, $J^{\ominus}$, $ClO_4^{\ominus}$, $NO_3^{\ominus}$, $BrO_3^{\ominus}$, $HSO_4^{\ominus}$, $H_2PO_3^{\ominus}$, $H_2PO_4^{\ominus}$, $BF_4^{\ominus}$, $B(Phenyl)_4^{\ominus}$, $PF_6^{\ominus}$, $SbF_6^{\ominus}$, $AsF_6^{\ominus}$, $SbCl_6^{\ominus}$, $SbCl_5F^{\ominus}$, $HCOO^{\ominus}$, $CH_3COO^{\ominus}$, $CCl_3COO^{\ominus}$, $CF_3COO^{\ominus}$, $CH_3SO_3^{\ominus}$, $CCl_3SO_3^{\ominus}$, $CF_3SO_3^{\ominus}$, Phenyl-$SO_3^{\ominus}$ oder p-Toluyl-$SO_3^{\ominus}$ handeln. In einer bevorzugten Ausführungsform stellt $X^{\ominus}$ Halogenid, $BF_4^{\ominus}$, $ClO_4^{\ominus}$, $CF_3SO_3^{\ominus}$ oder $PF_6^{\ominus}$ dar.

Eine bevorzugte Untergruppe von Iridiumkomplexen sind solche, in denen $X^{\ominus}$ in den Formeln VI und VIa $Cl^{\ominus}$, $Br^{\ominus}$, $J^{\ominus}$, $BF_4^{\ominus}$, $ClO_4^{\ominus}$, $CF_3SO_3^{\ominus}$ oder $PF_6^{\ominus}$ ist, und $R^4$ und $R^5$ zusammen für eine Bindung stehen.

Eine weitere bevorzugte Untergruppe von Iridiumkomplexen sind solche, in denen $X^{\ominus}$ in den Formeln VI und VIa $BF_4^{\ominus}$, $ClO_4^{\ominus}$, $CF_3SO_3^{\ominus}$ oder $PF_6^{\ominus}$ ist und $R^4$ und $R^5$ zusammen je für H stehen.

Y stellt bevorzugt ein Dien mit 6 bis 8 C-Atomen, deren Diengruppen insbesondere über 2 C-Atome verbunden sind, dar. In einer bevorzugten Ausführungsform steht Y für 1,5-Cyclooctadien, Norbornadien oder 1,5-Hexadien.

Besonders bevorzugte Iridiumkomplexe sind solche der Formeln

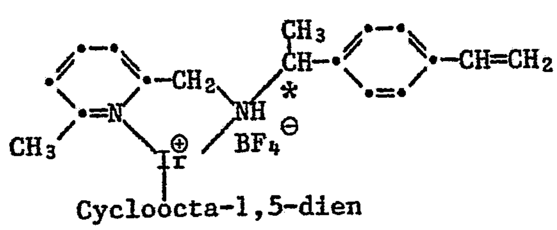

,

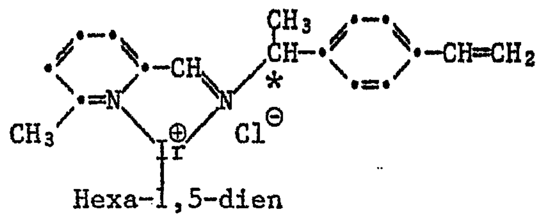

,

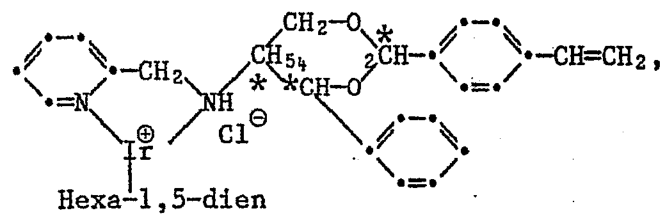

worin * für überwiegend R- oder S-Konfiguration bzw. 2R,4S,5S-Konfiguration steht.

Die Iridiumkomplexe der Formeln VI oder VIa können nach an sich bekannten Verfahren [siehe Inorganica Chimica Acta 73 (1983), S. 275 - 279] erhalten werden, indem man [$(Acetonitril)_2$(Y)]IrX, worin X und Y die zuvor angegebene Bedeutung haben, mit einer Verbindung der Formel I umsetzt. Die Herstellung des Acetonitrilkomplexes ist dort ebenfalls beschrieben. Die zur Herstellung des Acetonitrilkomplexes verwendeten Komplexe $[IrCl(Y)]_2$ sind z.B. durch die Umsetzung von Dichlorotetrakis(alken)diiridium(I) (Alken z.B. Cycloocten) mit einem Dien Y erhältlich. Die Iridiumkomplexe der Formeln VI und VIa können auch nach an sich bekannten Verfahren [siehe J. of Organom. Chem., 222, S. 323 - 329 (1981)] erhalten werden, indem man einen Diiridiumkomplex der Formel $[Ir(Y)Cl]_2$ mit einer Verbindung der Formel I umsetzt.

Die Umsetzungen werden im allgemeinen bei Temperaturen von -10 bis 30°C in einem inerten Lösungsmittel und unter Luftausschluss (Inertgasatmosphäre) durchgeführt. Geeignete inerte Lösungsmittel sind z.B. Kohlenwasserstoffe, wie Benzol, Toluol, Xylol, Petrolether, Hexan, Cyclohexan, Methylcyclohexan; und Ether wie z.B. Diethylether, Dibutylether, Tetrahydrofuran und Dioxan, sowie halogenierte Kohlenwasserstoffe, z.B. Chloroform, Methylenchlorid und Chlorbenzol. Zur Herstellung von Salzen der Formel VI oder VIa

mit Anionen einbasischer anorganischer oder organischer Säuren können die Salze, besonders die Chloride, der Formeln VI oder VIa, entweder direkt nach der Reaktion oder nach der Isolierung und Reinigung und dem erneuten Lösen in polaren Lösungsmitteln (z.B. Alkoholen, Ethern oder Ketonen, gegebenenfalls unter Zusatz von Wasser) mit einem Alkalisalz $M^{\oplus}X'^{\ominus}$ umgesalzt und danach isoliert werden. $X'^{\ominus}$ bedeutet ein von $X^{\ominus}$ verschiedenes Anion einer einbasischen anorganischen oder organischen Säure, $M^{\oplus}$ steht bevorzugt für Natrium.

Die Verbindungen der Formel I eignen sich zur Herstellung von Polymeren mit optisch aktiven Seitengruppen. Ein weiterer Gegenstand der Erfindung sind Homo- und Copolymere mit optisch aktiven Seitengruppen, enthaltend, bezogen auf die Polymeren,
a) 0,005 bis 100 Mol-% mindestens eines wiederkehrenden Strukturelementes der Formel VII

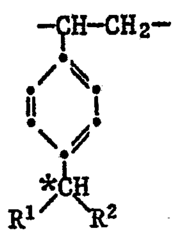
(VII),

worin $R^1$ $C_1$-$C_4$-Alkyl, Phenyl oder Benzyl bedeutet, $R^2$ einen Rest der Formeln II oder IIa

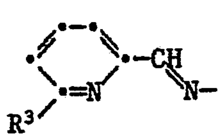
(II),
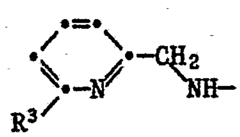
(IIa),

darstellt, worin $R^3$ für H oder $-CH_3$ steht, oder $R^1$ und $R^2$ zusammen ein Rest der Formel

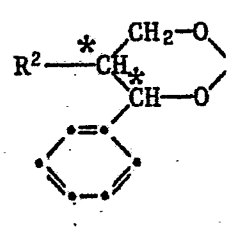

sind, worin $R^2$ die zuvor angegebene Bedeutung hat; und * für überwiegend R- oder überwiegend S-Konfiguration steht, und
b) 99,995 bis 0 Mol-% mindestens eines von einem olefinischen Comonomer abgeleiteten Strukturelementes, das von Komponente a) verschieden ist.

Für die Strukturelemente der Formel VII gelten die gleichen Bevorzugungen wie für die Verbindungen der Formel I. Die Strukturelemente der Formel VII sind bevorzugt in einer Menge von 0,05 bis 100, besonders 0,1 bis 50 und insbesondere 0,1 bis 10 Mol-%, und die Strukturelemente eines olefinischen Comonomers entsprechend in einer Menge von 99,95 bis 0, besonders 50 bis 99,9 und insbesondere 99,9 bis 90 Mol-% enthalten.

Das Strukturelement des olefinischen Comonomers entspricht bevorzugt der Formel VIII

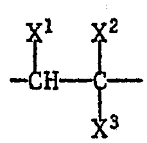

(VIII)

worin $X^1$ Wasserstoff, $X^2$ Wasserstoff, Chlor oder Methyl und $X^3$ Wasserstoff, Methyl, Chlor, -CN, $-CONR^7R^8$, worin $R^7$ und $R^8$ unabhängig H oder $C_1$-$C_{18}$-Alkyl sind, Phenyl, Methylphenyl, Methoxyphenyl, Cyclohexyl, -COO-Alkyl mit 1 - 12 C-Atomen im Alkyl, -COO-Phenyl, -COO-Alkyl-OH mit 2 - 6 C-Atomen im Alkyl, -OCO-Alkyl mit 1 - 4 C-Atomen im Alkyl, -OCO-Phenyl, Alkoxy mit 1 - 20 C-Atomen oder Phenoxy, oder $X^2$ Wasserstoff und $X^1$ und $X^3$ zusammen eine Gruppierung $-CO-NR^6-CO-$ oder je -COO-Alkyl mit 1 - 6 C-Atomen im Alkyl darstellen, wobei $R^6$ geradkettiges oder verzweigtes $C_1$-$C_{18}$-Alkyl, Cyclohexyl oder Phenyl bedeutet, das durch $C_1$-$C_6$-Alkyl, Halogen, Cyano, Nitro und/oder $C_1$-$C_3$-Alkoxy mono- oder disubstituiert sein kann.

Bevorzugt sind Copolymere, worin in Formel VIII $X^1$ für H steht, $X^2$ H oder $-CH_3$ bedeutet und $X^3$ Phenyl, Cyclohexyl, -COOAlkyl mit 1 - 12 C-Atomen im Alkyl, -COO-Hydroxyalkyl mit 2 bis 4 C-Atomen im Alkyl, oder $-CONR^7R^8$ darstellt, worin $R^7$ und $R^8$ unabhängig für H oder $C_{1-12}$-Alkyl steht.

Besonders bevorzugt sind Copolymere, worin in Formel VIII, $X^1$ und $X^2$ für H und $X^3$ für Phenyl, oder $X^1$ für H, $X^2$ für $-CH_3$ und $X^3$ für -COO Alkyl mit 1 bis 12 C-Atomen im Alkyl stehen.

$X^3$, $R^6$, $R^7$ und $R^8$ als Alkyl können linear oder verzweigt sein und enthalten bevorzugt 1 bis 12, besonders 1 bis 6 C-Atome. Die erfindungsgemässen Polymere können zusätzlich, bezogen auf die vorhandenen Monomeren, bis zu 60 Mol-% Strukturelemente eines zwei-oder mehrfach olefinisch ungesättigten Vernetzungsmittels enthalten. Geeignete mehrfach ungesättigte Vernetzungsmittel sind z.B. Divinylbenzole, Divinyltoluole, Divinylnaphthaline, Divinylxylole, Divinylethylbenzole, Divinylsebacat, Trivinylbenzole, Trivinylnaphthaline, Polyvinylanthracene; Ethylenglycoldiacrylat, Ethylenglycoldimethacrylat, N,N'-Methylendiacrylamid, N,N'-Methylendimethylacrylamid, N,N'-Ethylendiacrylamid, Polyvinylether von Ethylenglycol, Propantriol, Pentaerythritol und Resorcinol.

Das Vernetzungsmittel wird vorzugsweise in einer Menge von 0,1 - 10 Mol % eingesetzt. Bevorzugte Vernetzungsmittel sind Divinylbenzole. Durch geeignete Wahl der Comonomeren und/oder Vernetzungsmittel können die Eigenschaften der Polymeren den gewünschten spezifischen Anwendungen angepasst werden.

Die Polymeren können ein mittleres Molekulargewicht ($\bar{M}_w$) von 1 000 bis 5 000 000, vorzugsweise 5 000 bis 1 000 000 und besonders 5 000 bis 500 000 aufweisen (osmometrische Bestimmung).

Die erfindungsgemässen Polymeren können in bekannter Weise erhalten werden, indem man

a) 0,005 bis 100 Mol-% mindestens eines Monomers der Formel I und
b) 0 bis 99,995 Mol-% mindestens eines olefinischen Comonomers, das von Komponente a) verschieden ist,
polymerisiert.

Bevorzugt ist die radikalische Polymerisation. Dabei verwendet man zweckmässig etwa 0,01 bis 5 Gew.%, vorzugsweise 0,01 bis 1,5 Gew.%, bezogen auf das Gesamtgewicht der Monomeren und allfälliger Vernetzungsmittel, an sich bekannter Radikalinitiatoren, wie anorganische oder organische Peroxide oder Azoverbindungen, z.B. Wasserstoffperoxid, Kaliumperoxidsulfat, tert-Butylhydroperoxid, Di-tert-butylperoxid, Peressigsäure, Dibenzoylperoxid, Diacylperoxide, Cumolhydroperoxid, tert-Butylperbenzoat, tert-Alkylperoxidcarbonate und α,α'-Azoisobutyronitril. Die Reaktionstemperaturen für die radikalische Polymerisation liegen im allgemeinen zwischen etwa 30 und 100°C. Die Polymerisation kann in homogener Phase, z.B. in Substanz oder in Lösung, oder in heterogener Phase, d.h. als Fällungspolymerisation, Emulsions- oder Suspensionspolymerisation, erfolgen. Bevorzugt ist die Polymerisation in Lösung. Geeignete Lösungsmittel sind z.B. Toluol, N,N-Dimethylformamid, N,N-Dimethylacetamid, Acetonitril, Tetrahydrofuran und Dioxan.

Die erfindungsgemässen Polymeren können teilweise oder ganz mit Iridium (I)-Salzen komplexiert sein. Ein weiterer Gegenstand der Erfindung sind Polymere, die dadurch gekennzeichnet sind, dass mindestens ein Teil der Strukturelemente der Formel VII mit Iridium (I) komplexiert sind und den Formeln IX oder X entsprechen,

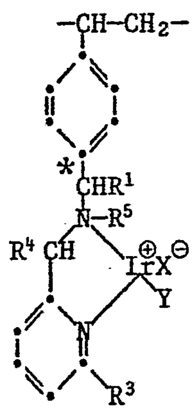 (IX) oder 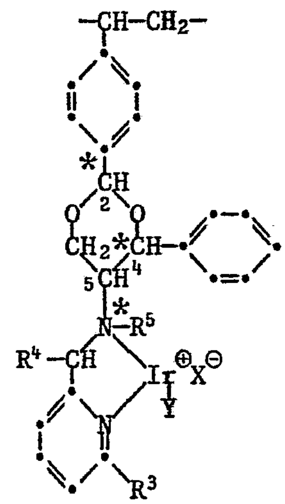 (X),

worin $R^1$ $C_1$-$C_4$-Alkyl, Phenyl oder Benzyl bedeutet, $R^3$ H oder $-CH_3$ sind, $R^4$ und $R^5$ je für H stehen oder zusammen eine Bindung darstellen, $X^{\ominus}$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten. Für $R^1$ bis $R^5$, $X^{\ominus}$ und Y und für die Polymeren gelten die zuvor angegebenen Bevorzugungen. Bevorzugt sind mindestens 50, besonders mindestens 90 Mol-% der vorhandenen Strukturelemente der Formel VII komplexiert.

Die komplexierten Polymeren können hergestellt werden, indem man
A) a) 0,005 bis 100 Mol-% mindestens eines Monomers der Formeln VI oder VIa gegebenenfalls zusammen mit einem Monomer der Formel I, und b) 0 bis 99,995 Mol-% mindestens eines olefinischen Comonomers, das von Komponente a) verschieden ist, polymerisiert, oder
B) dass man in Lösung ein erfindungsgemässes Homo- oder Copolymer mit Strukturelementen der Formel VII mit [(Acetonitril)$_2$(Y)]IrX oder mit Di-[μ-Chlorotetrakis(cycloocten)-diiridium (I)]Cl und einem Dien Y umsetzt, wobei X das Anion einer einbasischen anorganischen oder organischen Säure ist.

Die Durchführung des Verfahrens A) erfolgt im wesentlichen gleich wie die Polymerisation zur Herstellung der erfindungsgemässen nicht komplexierten Polymeren. Die Durchführung des Verfahrens B) erfolgt im wesentlichen gleich wie die Herstellung der Komplexe der Formeln VI oder VIa.

Die erfindungsgemässen Iridiumkomplexe und komplexierten Polymere eignen sich als homogene oder heterogene enantioselektive Katalysatoren für die Hydrierung von ungesättigten organischen Verbindungen mit bevorzugt mindestens einem prochiralen C-Atom. Insbesondere eignen sie sich als solche Katalysatoren für Transferhydrierungen, bevorzugt von Ketonen.

Ein weiterer Gegenstand der Erfindung ist die Verwendung von Iridiumkomplexen der Formeln VI und VIa als homogene enantioselektive Katalysatoren für die Transferhydrierung von prochiralen Ketonen mit sekundären Alkoholen sowie die Verwendung von erfindungsgemässen komplexierten Polymeren als homogene oder heterogene enantioselektive Katalysatoren für die Transferhydrierung von prochiralen Ketonen mit sekundären Alkoholen. Als sekundärer Alkohol ist besonders Isopropanol geeignet. Die Reaktion wird vorteilhaft unter Sauerstoffausschluss und bei erhöhter Temperatur (z.B. 50 - 150°C) durchgeführt. Zweckmässig wird der verwendete sekundäre Alkohol als Lösungsmittel eingesetzt. Die Katalysatorkonzentration beträgt bevorzugt $10^{-1}$ bis $10^{-5}$ Mol/l, bezogen auf das Reaktionsvolumen. Die Reaktion wird bevorzugt in Gegenwart einer Base, besonders NaOH, durchgeführt.

Die polymerisierbaren Verbindungen der Formel I und deren Polymere sind wertvolle optisch aktive Liganden für die Herstellung von chiralen, homogenen und heterogenen Katalysatoren mit verschiedenen komplexierenden Metallverbindungen. Die monomeren Metallkomplexe können überraschend polymerisiert werden. Ein besonderer Vorteil der heterogenen, polymeren Katalysatoren ist, dass sie leicht aus dem Reaktionsgemisch entfernt und wiederverwendet werden können. Die monomeren Metallkomplexe weisen als Katalysatoren eine gute Selektivität (optische Ausbeute) und hohe Ausbeuten auf, die von den heterogenen polymeren Katalysatoren sogar noch übertroffen wird. Die nachfolgenden Beispiele erläutern die Erfindung näher.

A) Herstellung von Ausgangsprodukten

Beispiel a): (2R,4S,5S)-(+)-5-Amino-2-(4-Vinylphenyl)-4-phenyl-1,3-dioxan

Zu einer Mischung von 6 g (0,024 Mol) (+)-2-Amino-1-phenyl-1,3-propandiol-hydrobromid, 25 ml p-Styrylaldehyd sowie einer Spatelspitze 2,6-Di-tert-butyl-p-kresol werden bei 5°C und unter Rühren 5,9 g (0,021 Mol) Phosphorpentoxid zugegeben. Die Suspension wird mittels Eiskühlung auf 20°C gehalten und 6 Stunden gerührt. Anschliessend wird bei 0°C mit 20 ml konzentrierter Kaliumcarbonat-Lösung versetzt und mit 50 ml Essigsäureethylester überschichtet. Unter Zugabe von 50 ml Eiswasser wird kräftig gerührt und dann das Gemisch durch Filtration von festen Bestandteilen befreit. Die wässrige Phase wird abgetrennt und zweimal mit Essigsäureethylester gewaschen. Die vereinigten organischen Phasen werden mit konzentrierter Natriumchlorid-Lösung (Sole) gewaschen, über Natriumsulfat getrocknet und bei ca. 2 kPa weitgehend vom Lösungsmittel befreit.

Der Rückstand wird in 250 ml Methanol aufgeschlämmt und mit 20 ml Essigsäure, einer Spatelspitze 2,6-Di-tert.-butyl-p-kresol sowie 42,3 g Acethydrazid-Trimethylammoniumchlorid (Girard-Reagens T) versetzt. Nach 15 Stunden Rühren bei Raumtemperatur wird das Reaktionsgemisch auf 1,5 l Eiswasser gegossen. Es werden portionsweise 33,3 g Natriumcarbonat zugegeben, anschliessend wird das Produkt viermal mit ca. 200 ml Essigsäureethylester extrahiert. Nach Trocknung über Natriumsulfat wird bei 1,5 kPa vom Lösungsmittel befreit. Das verbleibende gelbe Oel wird zur Reinigung an Kieselgel chromatographiert (Laufmittel: Methanol). Es werden 3,9 g (57 % der Theorie) öliges Produkt erhalten.
$[\alpha]_D^{20}$ = +35,7° (c = 1,143, Chloroform).

250 MHz-$^1$HFT-NMR ($CDCl_3$):
δ = 1,55 (s; 2H), 2,95 (m; 1H), 4,31 (m; 2H), 5,13 (m; 1H), 5,27 (m; 1H), 5,72 (s; 1H), 5,76 (m; 1H), 6,74 (m; 1H), 7,24 - 7,61 (m; 9H)ppm.

Beispiel b:
(R),(S)-1-[4-(2-Bromethyl)phenyl]-2-phenyl-ethylamin-hydrochlorid

Zu einer Lösung aus 20 g (0,066 Mol) 4-(2-Bromethyl)phenyl-benzylketon in 100 ml Dioxan werden eine Lösung aus 50 g (0,66 Mol) Ammoniumacetat in 200 ml Methanol, 2,7 g (0,43 Mol) Natriumcyanoborhydrid sowie ca. 100 mg 4-tert.-Butylbrenzkatechin zugegeben. Nach 48 Stunden Rühren bei Raumtemperatur wird mit 50 ml konzentrierter Salzsäure angesäuert (pH 2). Anschliessend werden bei ca. 2 kPa Methanol und Dioxan weitgehend abdestilliert und durch Essigsäureethylester ersetzt. Die Lösung wird zweimal mit je 100 ml Wasser gewaschen und bei ca. 2 kPa eingeengt. Der Rückstand wird mit Ethanol/Diethylether (1 : 1) aufgenommen. Der entstandene weisse Niederschlag wird abfiltriert und das Filtrat zu einem Oel eingeengt. Nach Zugabe von 50 ml Essigsäureethylester setzt Kristallisation des Produkts ein. Es wird abgesaugt und getrocknet. Ausbeute: 10,8 g (48 % d. Theorie) weisse Kristalle. Schmelzpunkt: 165 - 168°C/Zers.
Elementaranalyse:
Gef.: C 56,49; H 5,31; N 4,29; Br 23,02 %
Ber. für $C_{16}H_{19}NBrCl$ (340,69): C 56,44; H 5,62; N 4,11; Br 23,45 %.

(R),(S)-1-(4-Vinylphenyl)-2-phenylethylaminhydrochlorid

Aus 14,7 g (0,634 Mol) Natrium und 480 ml Ethanol wird eine Natriumalkoholat-Lösung hergestellt. Es werden 65 g (0,191 Mol) (R),(S)-1-[4-(2-Bromethyl)phenyl]-2-phenylethylamin-hydrochlorid zugegeben. Die gelbe Suspension wird 15 Minuten zum Rückfluss erhitzt und nach Abkühlen auf 1 l Eiswasser gegossen. Das Produkt wird dreimal mit je 500 ml Diethylether extrahiert. Die etherische

Phase wird mit Natriumsulfat getrocknet und mit Chlorwasserstoffgas versetzt. Nach Abdestillieren des Ethers wird das Rohprodukt aus Ethanol/Diethylether (1 : 1) umkristallisiert.
Ausbeute: 32,2 g (65 % d. Theorie) weisse Kristalle
Schmelzpunkt: 268 - 271°C/Zers.
Elementaranalyse:
Gef.: C 70,96; H 7,27; N 5,40; Cl 12,96; $H_2O$ 3,78 %
Ber. für $C_{16}H_{18}NCl \cdot 0{,}57\ H_2O$: C 71,18 %; H 7,14; N 5,19; Cl 13,13; $H_2O$ 3,78 %.

Bis-(S)-1-(4-Vinylphenyl)-2-phenylethylamin-L-tartrat

Aus (R),(S)-1-(4-Vinylphenyl)-2-phenylethylamin-hydrochlorid wird durch Behandeln mit Ionenaustauscher (Amberlyst A 26,OH-Form, Laufmittel : Methanol) die freie Base gewonnen. 23,1 g davon (0,103 Mol) werden in 500 ml Essigsäureethylester gelöst und mit 7,75 g (0,0502 Mol) L-(+)-Weinsäure, gelöst in 40 ml Ethanol, versetzt. Nach 48 Stunden bei ca. -15°C werden 25 g eines kristallinen Produkts erhalten, $[\alpha]_D^{20} = + 17{,}7°$ (MeOH, C = 0,926). Dieses wird sechsmal aus Ethanol umkristallisiert, wobei die optische Drehung auf den Wert $[\alpha]_D^{20} = + 134{,}5°$ (MeOH, c = 1,006) steigt.
Ausbeute: 3,8 g
Elementaranalyse:
Gef.: C 71,50; H 6,76; N 4,97; O 16,43 %;
Ber. für $C_{36}\ H_{42}\ O_6N_2$:
C 72,22; H 7,07; N 4,68; O 16,03 %.

(S)-1-(4-Vinylphenyl)-2-phenylethylamin

2,87 g (0,0048 Mol) des zuvor beschriebenen Tartrats werden in 50 ml Essigsäureethylester gelöst. Es werden 50 ml konzentrierte Natronlauge zugegeben. Die Essigesterphase wird abgetrennt und die Wasserphase noch zweimal mit je 25 ml Essigsäureethylester nachextrahiert. Die vereinigten organischen Phasen werden mit 25 ml Sole gewaschen, über Natriumsulfat getrocknet, mit einer Spatelspitze

Di-tert.-Butyl-p-kresol versetzt und bei ca. 2 kPa eingeengt. Der Rückstand wird im Kugelrohrofen bei 180°C und $9 \cdot 10^{-3}$ Pa destilliert. Ausbeute 2,0 g kristallisierendes Oel.
$[\alpha]_D^{20} = + 30,4°$ (Chloroform, c = 1,466).
Elementaranalyse:
Gef.: C 85.66, H 7,66; N 6,16 %
Ber. für $C_{16}H_{17}N$:
C 86,06; H 7,68; N 6,28 %.

Beispiel c:

(R)-1-(4-Vinylphenyl)ethylamin-L-malat

Zu einer Lösung aus 41,06 g (0,279 Mol) (R),(S)-1-(4-Vinylphenyl)ethylamin in 250 Methanol werden bei 0°C 37,4 g (0,279 Mol) L-(-)-Aepfelsäure gegeben. Nach 15 Stunden werden die ausgefallenen Kristalle (31,0 g) abgesaugt, die Mutterlauge wird unter Vakuum eingeengt, bis erneut Kristallisation einsetzt. Es werden weitere 15 g Produkt erhalten. Die Kristallfraktionen werden vereinigt und zweimal in je 250 ml Methanol unter Zusatz von ca. 50 mg Di-tert.-butyl-p-kresol umkristallisiert.
Ausbeute: 15,2 g weisses Kristallpulver,
$[\alpha]_D^{20} = + 2,10°$ ($H_2O$, c = 0,95).

(R)-1-(4-Vinylphenyl)ethylamin

14,0 g des Kristallpulvers [(R)-1-(4-Vinylphenyl)ethylamin-L-malat] werden in wenig Wasser gelöst und mit 100 ml Essigsäuremethylester überschichtet. Es wird mit 30 prozentiger Natronlauge alkalisch gestellt (pH > 10) und die Essigsäureethylester-Phase abgetrennt. Die Wasserphase wird 2x mit je 50 ml Essigsäureethylester nachgewaschen. Die organischen Phasen werden vereinigt, mit konzentrierter Natriumchlorid-Lösung (Sole) gewaschen, über Natriumsulfat getrocknet und unter Zusatz einer Spatelspitze Di-tert.-butyl-p-kresol

eingeengt. Der Rückstand wird im Hochvakuum destilliert.
Sdp. 54°C/$1.10^{-2}$ Pa, Ausbeute: 7,4 g farblose Flüssigkeit
$[\alpha]_D^{20}$ = + 36,2° (Chloroform, c = 0,968).

Beispiel d:

(S)-1-(4-Vinylphenyl)ethylamin-D-malat

Die bei den unter Beispiel c beschriebenen Kristallisationen anfallenden Mutterlaugen werden vereinigt und bei ca. 2 kPa weitgehend vom Lösungsmittel befreit. Analog dem in Beispiel c beschriebenen Verfahren wird das Amin freigesetzt und destilliert. Entsprechend Beispiel c wird das Amin (18,8 g) in 125 ml Methanol gelöst und mit D-(+)-Aepfelsäure (17,12 g) zur Reaktion gebracht. Das anfallende Kristallisat wird zweimal in Methanol umkristallisiert.
Ausbeute: 15,0 g weisses Kristallpulver,
$[\alpha]_D^{20}$ = 1,84° ($H_2O$, c = 1,25).

(S)-1-(4-Vinylphenyl)ethylamin

Analog Beispiel c werden 14 g (S)-1-(4-Vinylphenyl)ethylamin-L-malat mit Natriumhydroxid behandelt. Nach Aufarbeitung werden 7,5 g einer farblosen Flüssigkeit erhalten. Sdp. 53°C/$1\cdot10^{-2}$ Pa,
$[\alpha]_D^{20}$ = -34,9° (Chloroform, c = 0,986).

Beispiel 1: N-(6-Methyl-pyridin-2-carbaldehyd)-(R)-1-(4-vinylphenyl)ethylimin

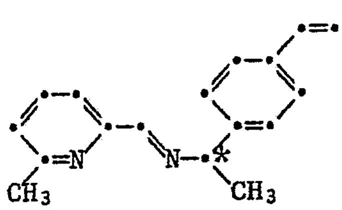

Eine Lösung aus 1,69 g (13,9 mmol) 6-Methylpyridin-2-aldehyd, 2,05 g (13,9 mmol), (R)-1-(4-Vinylphenyl)ethylamin, und 25 ml Ethanol wird 1 Stunde zum Rückfluss erhitzt. Das Lösungsmittel wird anschliessend bei ca. 3 kPa abdestilliert, der Rückstand im Kugelrohrofen bei 170°C und 0,11 Pa destilliert. Es werden 3,3 g (88 % d. Theorie)

eines Oels erhalten, das in der Kälte kristallisiert. Fp. ca 25°C, $[\alpha]_D^{20}$ = + 21,6° (c = 1,036, Chloroform).

Elementaranalyse:

Gef. C 81,71; H 7,25; N 11,02 %

Ber. für $C_{17}H_{18}N_2$ (250,35):

C 81,56; H 7,25; N 11,19 %.

Beispiel 2: N-(6-Methyl-pyridin-2-carbaldehyd)-(S)-1-(4-vinyl-phenyl)-2-phenyl-ethylimin

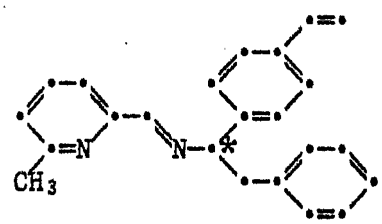

Eine Lösung aus 0,54 g (4,5 mmol)6-Methyl-pyridin-2-aldehyd, 1,0 g (4,5 mmol) (S)-1-(4-Vinylphenyl)-2-phenyl-ethylamin sowie 10 ml Benzol wird bei Raumtemperatur und ca. 3 kPa eingeengt. Es werden erneut 10 ml Benzol zugegeben, um restliches Reaktionswasser zusammen mit Benzol bei ca. 3 kPa abzudestillieren. Der Vorgang wird noch 2x wiederholt.

Nach vollständigem Entfernen des Lösungsmittels bei ca. 1,5 Pa werden 1,5 g (97 % d. Theorie) weisse Kristalle erhalten.

Elementaranalyse:

Gef. C 84,55; H 6,74; N 8,32 %

Ber. für $C_{23}H_{22}N_2$ (326,45): C 84,62; H 6,79; N 8,58 %

Beispiel 3: N-(Pyridin-2-carbaldehyd)-(2R,4S,5S)-5-imino-2-(4-vinyl-phenyl)-4-phenyl-1,3-dioxan

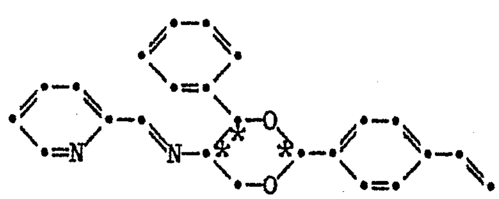

Eine Lösung aus 1,60 g (15,0 mmol) 2-Pyridinaldehyd, 4.25 g

(15,1 mmol) (2R,4S,5S)-5-Amino-2-(4-vinylphenyl)-4-phenyl-1,3-dioxan in 50 ml Benzol wird bei 40°C und ca. 3,5 Pa eingeengt. Es werden erneut 50 ml Benzol zugegeben, um restliches Reaktionswasser zusammen mit Benzol im Vakuum abzudestillieren. Der Rückstand wird mit 20 ml Benzol aufgenommen, durch Filtration von festen Bestandteilen befreit und mit Petrolether versetzt. Dabei kristallisiert das Produkt. Es wird abgesaugt und bei 20°C/1 Pa getrocknet. Rohausbeute; 3,8 g beiges Pulver (66 % d. Theorie). Nach Kristallisieren aus 15 ml Ethanol werden 2,3 g weisse Kristalle erhalten.

Fp. 113 - 114°C,

$[\alpha]_D^{20}$ = + 196,0° (c = 1,108, Chloroform)

Elementaranalyse

Gef.: C 77,10; H 6,13; N 7,20; O 9,09 %

Ber. für $C_{24}H_{22}N_2O_2$ (370,45):

C 77,81; H 5,99; N 7,56; O 8,64 %.

Beispiel 4: 2-{N-[(R)-1-(4-Vinylphenyl)ethyl]aminomethyl}-6-methylpyridin

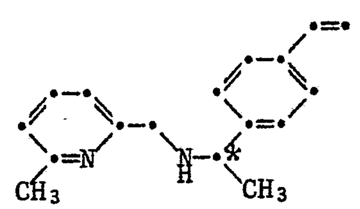

Zu einer Mischung aus 5 ml trockenem Tetrahydrofuran und 0,42 g Lithiumalanat wird bei 0°C und unter Feuchtigkeitsausschluss eine Lösung aus 1 g N-(6-Methyl-pyridin-2-carbaldehyd)-(R)-1-(4-vinylphenyl)ethylimin (Beispiel 1) und 5 ml trockenem Tetrahydrofuran zugetropft. Es wird anschliessend 4 Stunden ohne Kühlung gerührt, dann bei 0°C mit 1 ml konzentrierter Natriumsulfatlösung hydrolysiert. Der Niederschlag wird abfiltriert und mit Essigsäureethylester gewaschen. Die Wasserphase des Filtrats wird abgetrennt, mit Essigsäureethylester nachgewaschen und die vereinigten organischen Phasen über Natriumsulfat getrocknet. Nach Abdampfen des Lösungsmittels bei Raumtemperatur und ca. 1,5 Pa wird der Rückstand im Kugelrohrofen bei 250°C Ofentemperatur und 0,01 Pa destilliert. Es werden 0,63 g (63 % d. Theorie) eines gelben Oels erhalten.

$[\alpha]_D^{20}$ = +52,9 (0,926, Chloroform).
250 MHz-$^1$HFT-NMR ($CDCl_3$):
δ = 1.41 (d; 3H), 2,26 (breit; 1H), 2,53 (s; 3H), 3,70 (s; 2H), 3,82 (q; 1H), 5,21 (m; 1H), 5,72 (m; 1H), 6,71 (m; 1H), 6,97 - 7,50 (m; 7H)ppm.

Beispiel 5: 2-[{N-[(S)-1-(4-Vinylphenyl)-2-phenyl-ethyl]amino-methyl}-6-methyl-pyridin

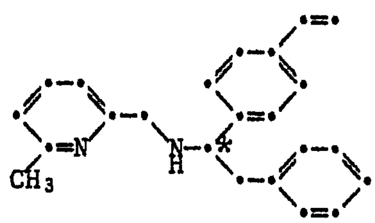

Entsprechend Beispiel 4 werden 1,15 g N-(6-Methyl-pyridin-2-carbaldehyd)-(S)-1-(4-vinylphenyl)-2-phenyl-ethylimin mittels 0,2 g Lithiumalanat reduziert. Es werden 0,9 g (83 % d. Theorie) eines farblosen Oels erhalten, welches an Kieselgel chromatographiert wird (Laufmittel: Essigsäureethylester). Nach Einengen der 2. Fraktion (Hauptfraktion) und Trocknen des verbleibenden Oels im Vakuum (ca. 1,2 Pa) werden 0,6 g Produkt als farbloses Oel erhalten.
$[\alpha]_D^{20}$ = -15,4° (c = 1,414, Chloroform)
Elementaranalyse
Gef.: C 84,21 H 7,20 N 9,03 %
Ber. für $C_{23}H_{24}N_2$ (328,46):
C 84,11; H 7,37; N 8,53 %.

Beispiel 6: 2-{N-[2R,4S,5S)-2-(4-Vinylphenyl)-4-phenyl-1,3-dioxan-5-yl]aminomethyl}pyridin

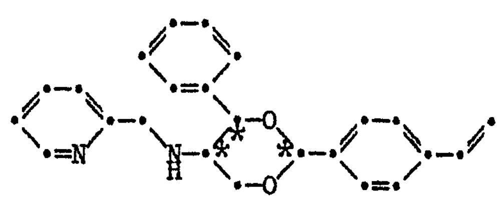

Entsprechend Beispiel 4 wird 1,0 g N-(Pyridin-2-carbaldehyd)-(2R,4S,5S)-5-imino-2-(4-vinylphenyl)-4-phenyl-1,3-dioxan (Beispiel 3) mittels 0,16 g Lithiumalanat reduziert. Nach Chromatographie an Kieselgel (Laufmittel: Essigsäureethylester) werden 0,55 g eines gelblichen Oels erhalten.
$[\alpha]_D^{20}$ = + 58,1 (c = 1,080 Chloroform)

250 MHz-$^1$HFT-NMR ($CDCl_3$):
δ = 2,28 (breit; 1H), 2,71 (d; 1H), 3,68-3,93 (m; 2H), 4,10 - 4,49 (m; 2H), 5,12 (d; 1H), 5,26 (m; 1H), 5,74 (s; 1H), 5,77 (m; 1H), 6,72 (m; 1H), 6,76 - 8,35 (m; 13 H).

Beispiel 7: [Ir(N,N)(HD)Cl], N,N: Verbindung gemäss Beispiel 1
Unter Argonschutzgas werden 0,600 g (0,68 mmol) Di-μ-chlorotetrakis-(cycloocten)diiridium (I) in 50 ml Benzol gelöst. Bei 10°C werden 4,4 ml 1,5-Hexadien (HD) zugegeben. Nach 30 Minuten Rühren bei Raumtemperatur werden 375 mg (1,5 mmol) N-(6-Methyl-pyridin-2-carbaldehyd)-(R)-1-(4-vinylphenyl)ethylimin (Beispiel 1) zugegeben.

Nach 1 Stunde Rühren bei Raumtemperatur werden unter Luftausschluss 100 ml n-Hexan zugegeben. Im Verlauf von 2 Stunden kristallisiert bei 0°C das Produkt aus. Es wird unter Argonatmosphäre abgesaugt, mit n-Hexan gewaschen und 3 Stunden bei 1,3 Pa getrocknet. Ausbeute: 0,500 g (66 % d. Theorie) beiges Pulver.
Elementaranalyse:
Gef. C 48,05; H 5,16; N 4,99; Ir 34,3 %
Ber. für $C_{23}H_{28}N_2IrCl$ (560.16):
C 49,32; H 5,04; N 5,00; Ir 34,31 %.

Beispiel 8: [Ir(N,N)(HD)J], N,N: Verbindung gemäss Beispiel 3
Unter Argonschutzgas werden 0,492 g (0,55 mmol) Di-μ-chlortetrakis(cycloocten)diiridium (I) in 80 ml Aceton gelöst. Bei 5°C werden 3,4 ml 1,5-Hexadien (HD) zugegeben. Es wird 10 Minuten auf 35°C erwärmt, anschliessend bei 0°C eine Lösung aus 0,380 g (2,5 mmol) Natriumjodid in 12 ml Wasser unter Luftausschluss zugegeben, unmittelbar darauf eine Lösung aus N-(Pyridin-2-carbaldehyd)-(2R,4S,5S)-5-imino-2-(4-vinylphenyl)-4-phenyl-1,3-dioxan (Beispiel 3) in 4 ml Aceton. Nach 1 Stunde wird auf die Hälfte des

Volumens eingeengt. Im Verlauf einer Stunde kristallisiert das Produkt bei 0°C aus. Es wird unter Schutzgas abgesaugt, mit ca. 40 ml Wasser gewaschen und 8 Stunden bei 0,13 Pa über Phosphorpentoxid getrocknet. Ausbeute: 0,520 g (67 % d. Theorie) dunkelblaue Kristalle.

Elementaranalyse:

Gef. C 46,59; H 4,16; N 3,60; J 16,31;

Ber. für $C_{30}H_{32}N_2O_2Ir$ (771,71): C 46,69; H 4,18, N 3,63; J 16,44%.

Beispiel 9: [Ir(N,N)(COD)]$BF_4$, NN: Verbindung gemäss Beispiel 4, COD: Cycloocta-1,5-dien

Unter Argonschutzgas werden 0,469 g (1,0 mmol) [Bis(acetonitril)cycloocta-1,5-dien]iridiumtetrafluoroborat in 15 ml Dichlormethan gelöst. Bei Raumtemperatur und unter Rühren wird eine Lösung aus 5 ml Dichlormethan und 0,252 g (1.0 mmol) 2-{N-[(R)-1-(4-Vinylphenyl)ethyl]aminomethyl}-6-methyl-pyridin (Beipiel 4) zugetropft. Nach 1 Stunde wird bei ca. 600 Pa auf etwa ein Drittel des Volumens eingeengt. Es werden 60 ml Diethylether zugegeben, wobei das Produkt innerhalb 2 Stunden als fester Niederschlag ausfällt. Das Produkt wird unter Argonatmosphäre abgesaugt, dreimal mit Diethylether gewaschen und 15 Stunden bei 0,1 Pa getrocknet. Ausbeute 0,588 g (92 % d. Theorie) ockergelbe Kristalle.

Elementaranalyse:

Gef.: C 45,07; H 5,12; N 4,27; Ir 28,5 %

Ber. für $C_{25}H_{32}N_2IrBF_4 \cdot 1,5\ H_2O$ (666,57):

C 45,05; H 5,29; N 4,20; Ir 28,83 %.

Beispiel 10: [Ir(N,N)(COD)]$BF_4$, N,N: Verbindung gemäss Beispiel 5

Analog Beispiel 9 werden 0,469 g (1,0 mmol) Bis(acetonitril(cycloocta-1,5-dien)iridium-tetrafluoroborat mit 0,316 g (1.0 mmol) 2-{N-[(S)-1-(4-Vinylphenyl)-2-phenyl-ethyl]aminomethyl}-6-methylpyridin (Beispiel 5) zur Reaktion gebracht. Nach entsprechender Aufarbeitung werden 0,641 g (91 % d. Theorie) gelbe Kristalle erhalten.

Elementaranalyse:

Gef.: C 49,80; H 5,19; N 3,82; Ir 24,9 %; F 10,32 %

Ber. für $C_{31}H_{36}N_2IrBF_4 \cdot 2\ H_2O$ (751,68):

C 49,53; H 5,36; N 3,79; Ir 25,57 %; F 10,11 %.

Beispiel 11: [Ir(N,N)(COD)]$BF_4$, N,N: Verbindung gemäss Beispiel 6

Analog Beispiel 9 werden 0,260 g (0,605 mmol) Bis(acetonitril)cycloocta-1,5-dien)iridium-tetrafluoroborat mit 0,225 g (0,605 mmol) 2-{N-[(2R,4S,5S)-2-(4-Vinylphenyl)-4-phenyl-1,3-dioxan-5-yl]aminomethyl}pyridin (Beispiel 6) zur Reaktion gebracht. Nach entsprechender Aufarbeitung werden 0,380 g (79 % der Theorie) orange-braune Kristalle erhalten.

Elementaranalyse:

Gef. C 48,36; H 4,93; N 3,74; F 10,18; Ir 24,5 %

Ber. für $C_{32}H_{40}N_2O_4IrBF_4 \cdot 2\ H_2O$ (795,69): C 48,30; H 5,07; N 3,52; F 9,55; Ir 24,16 %.

Beispiel 12: Copolymer aus [Ir(N,N)(HD)Cl] gemäss Beispiel 7 (2 Mol %) Methacrylsäure-2-ethylhexylester (96 Mol %) und technischem Divinylbenzol (2 Mol %)

30,0 mg (0,0536 mmol) Komplex, hergestellt gemäss Beispiel 7, 6,97 mg (0,0536 mmol) techn. Divinylbenzol (techn. DVB) und 509,8 mg (2,571 mmol) frisch destillierter Methacrylsäure-2-ethylhexylester werden in 7 ml Tetraydrofuran gelöst. In einer mit Argon gespülten Ampulle wird die dunkel rotbraune Lösung mit 3,3 mg Azobisisobutyronitril (AIBN), gelöst in 1 ml Tetrahydrofuran, gemischt. Es wird unter Luftausschluss auf 70°C erhitzt und nach 24 Stunden, dann nach weiteren 18 Stunden je die gleiche Menge AIBN zugegeben. Nach weiteren 30 Stunden Polymerisation bei 75°C wird eine im Durchlicht leuchtend rote feinteilige Emulsion erhalten. Der Restmonomerengehalt an Methacrylsäure-2-ethylhexylester wird mittels Gaschromatographie (CW-20 M) auf 7 % der ursprünglich eingesetzten Menge bestimmt.

Das Volumen wird auf 3,5 ml eingeengt und die Emulsion für die katalytische Anwendung verwendet. Die Katalysatorkapazität wird mit $1,148 \cdot 10^{-5}$ Mol Wiederholungseinheiten Iridiumkomplex pro 1 ml Emulsion bestimmt.

Beispiel 13: Copolymer aus N,N-Ligand (gemäss Beispiel 1, 5 Mol %) und Styrol (95 Mol %)

0,2504 g (1 mmol) N-(6-Methyl-pyridin-2-carbaldehyd)-(R)-1-vinyl-phenyl)ethylimin(N,N-Ligand) und 1,979 g (19,0 mmol) frisch destilliertes Styrol werden in 5 ml Benzol gelöst. In einer mit Argon gespülten Ampulle wird die Lösung mit 24,6 mg AIBN gemischt und 20 Stunden auf 70°C erhitzt. Die entstandene viskose Lösung wird um ca. 1/3 des Volumens mit Benzol verdünnt. Durch Eingiessen in 250 ml Methanol wird ein weisses Pulver erhalten. Es wird in Tetrahydrofuran gelöst und aus Methanol umgefällt. Es wird durch Zentrifugieren vom überstehenden Fällungsmittel abgetrennt und 15 Stunden bei 40°C und 0,12 Pa getrocknet. Ausbeute 1,03 g
$[\alpha]_D^{23}$= +0,44° (c = 4,966, Chloroform).
Elementaranalyse
Gef.: C 90,43; H 7,70; N 1,39 %,
Ber. für 0,056 ($C_{17}H_{18}N_2$) + 0,944 ($C_8H_8$): C 90,92; H 7,68; N 1,40 %
Ligandenkapazität: 0,499 mmol/g.

Beispiel 14: Copolymer aus [Ir(N,N)(HD)Cl](5 Mol %) und Styrol (95 Mol %) durch polymeranaloge Umsetzung an dem Polymerligand gemäss Beispiel 13

Unter Argonschutzgas werden 0,0893 g Di-µ-chlorotetrakis (cycloocten)diiridium(I) $BF_4$ in 5 ml Benzol gelöst. Bei 10°C werden 1,2 ml 1,5-Hexadien (HD) zugegeben. Nach 30 Minuten Rühren bei Raumtemperatur wird eine Lösung aus 0,4000 g Polymer, hergestellt nach Beispiel 13, und 5 ml Benzol zugetropft. Es entsteht eine dunkelrote Lösung, die nach 1 Stunde Rühren unter Luftausschluss auf ca. 1/3 des Volumens eingeengt und direkt zur katalytischen Transferhydrierung verwendet wird. Die Katalysatorkapazität wird mit $1{,}766 \cdot 10^{-5}$ Mol Wiederholungseinheiten pro 1 ml Lösung bestimmt.

Beispiel 15: Copolymer aus [Ir(N,N(COD)]$BF_4$ (gemäss Beispiel 9) (2 Mol %), Methacrylsäure-2-ethylhexylester (96 Mol %) und 1,4-Divinylbenzol (2 Mol %)

95,9 mg (0,15 mmol) Komplex, hergestellt gemäss Beispiel 9, 19,5 mg (0,15 mmol) 1,4-Divinylbenzol (1,4-DVB) und 1,428 g (7,2 mmol) frisch destillierter Methacrylsäure-2-ethylhexylester werden in 12 ml Tetrahydrofuran gelöst. In einer mit Argon gespülten Ampulle wird die ockerfarbene Lösung mit 9,23 mg AIBN, gelöst in 0,2 ml Tetrahydrofuran, gemischt. Es wird unter Luftausschluss auf 70°C erhitzt und nach 65 Stunden erneut die gleich Menge AIBN zugegeben. Nach weiteren 48 Stunden bei 70°C wird eine viskose, feinteilige Emulsion mit heller rotbrauner Farbe erhalten. Das Volumen wird auf 10 ml eingestellt, wobei die Katalysatorkapazität mit $1{,}42 \cdot 10^{-5}$ Mol Wiederholungseinheiten Iridiumkomplex pro 1 ml Emulsion bestimmt wird.

Beispiel 16: Copolymer aus [Ir(N,N)(COD)]$BF_4$ (gemäss Beispiel 10)] (2 Mol %), Methacrylsäure-2-ethylhexylester (96 Mol %) und 1,4-DVB (2 Mol %)

Analog Beispiel 15 werden 107,3 mg (0,15 mmol) Komplex, hergestellt gemäss Beispiel 10, 19,5 mg (0,15 mmol) 1,4-DVB und 1,428 g (7,2 mmol) Methacrylsäure-2-ethylhexylester in 12 ml Tetrahydrofuran copolymerisiert.
Es wird eine viskose, feinteilige Emulsion mit ockergelber Farbe erhalten.

Beispiel 17: Copolymer aus [Ir(N,N)(COD)$BF_4$(gemäss Beispiel 11) (2 Mol %), Methacrylsäure-2-ethylhexylester (96 Mol %) und 1,4-DVB (2 Mol %)

Analog Beispiel 15 werden 113,9 mg (0,15 mmol) Komplex, hergestellt gemäss Beispiel 11, 19,5 mg (0,15 mmol) 1,4-DVB und 1,428 g (7,2 mmol) Methacrylsäure-2-ethylhexylester in 12 ml Tetrahydrofuran copolymerisiert.

Es wird eine viskose orangebraune Emulsion erhalten. Die Konzentration wird wie in Beispiel 15 beschrieben eingestellt.

Beispiel 18: Copolymer aus N,N-Ligand (gemäss Beispiel 3, 2 Mol %), Methacrylsäure-2-ethylhexylester (96 Mol %) und 1,4-DVB (2 Mol %)

Analog Beispiel 13 werden 111,1 mg (0,3 mmol) N-(Pyridin-2-carbaldehyd)-(2R,4S,5S)-5-imino-2-(4-vinylphenyl)-4-phenyl-1,3-dioxan, 39,1 mg (0,3 mmol) 1,4-DVB und 2,856 g (0,0144 Mol) Methacrylsäure-2-ethyl-hexylester in 15 ml Tetrahydrofuran copolymerisiert. Nach 22 Stunden wird ein farbloses Oel erhalten, welches durch zweimaliges Versetzen mit Benzol und Abdampfen von Tetrahydrofuran bei ca. 3 kPa umgequollen wird. Das Produkt gelangt ohne Isolierung zur weiteren Umsetzung (Beispiel 19).

Beispiel 19: Copolymer aus [Ir(N,N) (HD) Cl] (2 Mol %), Methacrylsäure-2-ethylhexylester (96 Mol %) und 1,4-DVB (2 Mol %) durch polymeranaloge Umsetzung an den Polymerliganden gemäss Beispiel 18

Analog Beispiel 3 werden 0,130 g Di-μ-chlorotetrakis(cycloocten)diiridium (I) mit 1,5 Hexadien und dem Polymer gemäss Beispiel 18 umgesetzt. Nach 16 Stunden wird ein tiefblaues, im Durchlicht rotviolettes Gel erhalten. Es wird durch dreimaliges Versetzen mit Isopropanol unter Luftausschluss und Abdampfen vom Lösungsmittel gequollen. Es wird ein braunes Gel erhalten, das direkt zur katalytischen Anwendung gelangt. Eine Probe wird 15 Sekunden bei 0,1 Pa getrocknet.

Elementaranalyse:

Gef. C 71,48; H 10,73; N 0.3; Ir 1,71 %,

Ber. für 0,02 ($C_{30}H_{32}N_2O_2IrCl$) + 0,02 ($C_{10}H_{10}$) + 0,96 ($C_{12}H_{22}O_2$)
C 71,63; H 10,72; N 0,27; Ir 1,86 %.

Beispiel 20: Copolymer aus [Ir(N,N) (COD)] $BF_4$ gemäss Beispiel 9 (2 Mol %), Methacrylsäure-tert-butylester (96 Mol %) und 1,4-Divinylbenzol (2 Mol %)

Analog Beispiel 15 werden 128 mg (0,2 mmol) Komplex, hergestellt gemäss Beispiel 9, 26 mg (0,2 mmol) 1,4-DVB und 1,365 g (9,6 mmol) Methacrylsäure-tert-butylester in 12 ml Tetrahydrofuran copolymerisiert.

Es wird eine viskose, feinteilige Emulsion mit orange-brauner Farbe erhalten. Das Volumen wird auf 10 ml eingestellt.

C) Anwendungsbeispiele

Beispiel 21: 70,0 mg des gemäss Beispiel 7 hergestellten Komplexes werden unter Sauerstoffausschluss (Argonatomosphäre) in 31 ml Isopropanol gelöst. Nach 1 Stunde Rühren bei 60°C werden 5,0 ml 0,1 normale Natriumhydroxid-Lösung zugegeben. Es wird eine weitere Stunde bei 60°C gerührt und anschliessend eine Lösung aus 31 ml Isopropanol und 1,85 g 1-Phenylbutanon unter Sauerstoffausschluss hinzugefügt. Das molare Verhältnis Substrat zu Katalysator beträgt somit [S]/[Kat.] = 100, die Katalysatorkonzentration [Kat.] = $2 \cdot 10^{-3}$ Mol/l.

Nach 16 Stunden bei 60°C wird gaschromatographisch (10 % CW - 20 M, 185°C, isotherm) eine Ausbeute an 1-Phenylbutanol von 81,1 % bestimmt.

Zur Bestimmung des Enantiomerengehalts nach Mosher (J.A. DALE, D.L. DULL and H.S. MOSHER, J. Org. Chem. 34 (1969) 2543) wird eine Probe (ca. 0,5 ml) weitgehend vom Lösungsmittel befreit und bei 0°C mit 50 µl optisch reinem α-Methoxy-α-trifluormethylphenylacetylchlorid sowie 0,25 ml trockenem Pyridin versetzt. Nach 15 Minuten wird 30 Minuten auf 70°C erwärmt, nach Abkühlen mit 3 ml 10 prozentiger Zitronensäurelösung versetzt und mit Diethylether die diastereomeren Ester extrahiert.

Gaschromatographisch (Kapillarsäule CW 20, 190°C, isotherm) wird ein Enantiomerenüberschuss an (R)-1-Phenylbutanol von ee = 26,0 % bestimmt.

Beispiel 22: Entsprechend Beispiel 21 wird der Komplex nach Beispiel 8 zur katalytischen enantioselektiven Transferhydrierung von 1-Phenylbutanon verwendet.

Nach 19 Stunden 30 Minuten beträgt die Ausbeute 23,6 % an 1-Phenylbutanol, der Enantiomerenüberschuss ee = 60,3 % (S).

Beispiel 23: Entsprechend Beispiel 21 wird der Komplex nach Beispiel 9 zur katalytischen, enantioselektiven Transferhydrierung von 1-Phenylbutanon verwendet.

Nach 2 Stunden 50 Minuten beträgt die Ausbeute 97,3 % an 1-Phenylbutanol, der Enantiomerenüberschuss ee = 48,7 % (R).

Beispiel 24: Entsprechend Beispiel 21 wird der Komplex nach Beispiel 11 zur katalytischen, enantioselektiven Transferhydrierung von 1-Phenylbutanon verwendet.

Nach 20 Stunden beträgt die Ausbeute 75,2 % an 1-Phenylbutanol, der Enantiomerenüberschuss ee = 58,1 % (S).

Beispiel 25: 3,5 ml der nach Beispiel 12 erhaltenen Emulsion werden unter Sauerstoffausschluss (Argonatmosphäre) zu 15 ml Isopropanol gegeben. Nach 1 Stunde Rühren bei 60°C werden 1,61 ml 0,1 normale Natriumhydroxid-Lösung zugegeben, wobei das zuvor feinteilig dispergierte Polymer zu grösseren Partikeln agglomeriert. Es wird eine weitere Stunde bei 60°C gerührt und anschliessend eine Lösung aus 5 ml Isopropanol und 0,60 g 1-Phenylbutanon unter Sauerstoffausschluss hinzugefügt. Das molare Verhältnis Substrat zu Katalysator beträgt somit [S]/[Kat.] = 100.

Nach 19 Stunden bei 60°C wird gaschromatographisch (10 % CW - 20 M, 185°C, isotherm) eine Ausbeute an 1-Phenylbutanol von 79,7 % bestimmt.

Zur Bestimmung des Enantiomerengehalts nach Mosher wird eine Probe (ca. 0,5 ml) weitgehend vom Lösungsmittel befreit und bei 0°C mit 50 µl optisch reinem α-Methoxy-α-trifluormethylphenylacetylchlorid sowie 0,25 ml trockenem Pyridin versetzt. Nach 15 Minuten wird 30 Minuten auf 70°C erwärmt, nach Abkühlen mit 3 ml 10 prozentiger Zitronensäurelösung versetzt und mit Diethylether die diastereomeren Ester extrahiert.

Gaschromatographich (Kapillarsäule CW 20, 190°C, isotherm) wird ein Enantiomerenüberschuss an (R)-1-Phenylbutanol von ee = 66,2 % (R) bestimmt.

Beispiel 26: Entsprechend Beispiel 25 werden 5,5 ml der nach Beispiel 14 erhaltenen Lösung zur katalytischen enantioselektiven Transferhydrierung von 1-Phenylbutanon verwendet. Der Katalysator agglomeriert unter den Reaktionsbedingungen der Aktivierung zu einer kompakten Masse.

Nach 41 Stunden beträgt die Ausbeute 12,4 % an 1-Phenylbutanol, der Enantiomerenüberschuss ee = 42,2 % (R).

Beispiel 27: Entsprechend Beispiel 25 werden 0,8 ml der nach Beispiel 15 erhaltenen Emulsion zur katalytischen, enantioselektiven Transferhydrierung von 1-Phenylbutanon verwendet. Es wird jedoch das Konzentrationsverhältnis [S]/[Kat] = 1000 gewählt. Der heterogene Katalysator ist während der Reaktion feinteilig dispergiert.

Nach 6 Stunden beträgt die Ausbeute 91,9 % an 1-Phenylbutanol, der Enantiomerenüberschuss ee = 85,3 % (R).

Beispiel 28: Entsprechend Beispiel 25 werden 70 ml der nach Beispiel 15 erhaltenen Emulsion zur katalytischen, enantioselektiven Transferhydrierung von 1-Phenylbutanon verwendet. Es wird das Konzentrationsverhältnis [S]/[Kat.] = 100 gewählt.

Nach 2 Stunden 50 Minuten beträgt die Ausbeute 96,1 % an 1-Phenylbutanol, der Enantiomerenüberschuss ee = 78,7 % (R).

Beispiel 29: Der gemäss Beispiel 28 verwendete Katalysator wird wiedergewonnen, indem er von der überstehenden Lösung abgetrennt und das zurückbleibende braune Gel zweimal mit Isopropanol unter Luftausschluss gewaschen wird. Nach dem Dekantieren wird das Gel mit 30 ml Isopropanol dispergiert und bei 60°C mit 3,98 ml 0,1 normaler Natriumhydroxid-Lösung versetzt. Nach einer Stunde bei 60°C werden 1,48 g 1-Phenylbutanon in 30 ml Isopropanol zugegeben. Des weiteren wird wie in Beispiel 25 beschrieben verfahren.

Nach 2 Stunden beträgt die Ausbeute 94,5 % an 1-Phenylbutanol, der Enantiomerenüberschuss ee = 82,6 % (R).

Die Vorgänge des Wiedergewinnens, Reinigens und Wiedereinsetzens des Katalysators werden noch zweimal wiederholt.

Die Ausbeuten an 1-Phenylbutanol betragen nach 3 Stunden 25 Minuten 94,6 % bzw. nach 3 Stunden 45 Minuten 90,9 %, die Enantiomerenüberschüsse ee = 80,8 % (R) bzw. ee = 78,8 % (R).

Beispiel 30: Entsprechend Beispiel 25 werden 2,2 ml der nach Beispiel 15 erhaltenen Emulsion zur katalytischen enantioselektiven Transferhydrierung von 1,4-Diphenylbutanon verwendet.

Nach 6 Stunden 30 Minuten beträgt die Ausbeute 94,5 % an 1,4-Diphenylbutanol. Der Enantiomerenüberschuss wird aus dem $^{19}F$-NMR-Spektrum der diastereomeren Ester nach Mosher mit ee = 85,7 % bestimmt.

Beispiel 31: Analog Beispiel 25 werden 7,0 ml der nach Beispiel 17 erhaltenen Emulsion zur katalytischen, enantioselektiven Transferhydrierung von 1-Phenylbutanon verwendet. Der heterogene Katalysator ist während der Reaktion grobteilig dispergiert.

Nach 20 Stunden beträgt die Ausbeute 85,8 % an 1-Phenylbutanol, der Enantiomerenüberschuss ee = 78,7 % (S).

Beispiel 32: Entsprechend Beispiel 25 werden 5 ml der nach Beispiel 20 erhaltenen Emulsion zur katalytischen enantioselektiven Transferhydrierung von 1-Phenylbutanon verwendet. Es wird das Konzentrationsverhältnis [S]/[Kat] = 100 gewählt. Nach Beendigung der Aktivierungszeit wird die Reaktionstemperatur auf 24°C gesenkt.

Nach 45 Stunden beträgt die Ausbeute 95,2 % an 1-Phenylbutanol, der Enantiomerenüberschuss ee = 90,6 % (R).

Patentansprüche für die Vertragsstaaten: DE, GB, FR, IT, NL, SE, CH, LI, BE

1. Optisch aktive Verbindungen der Formel I

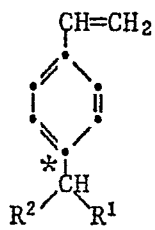

(I),

worin $R^1$ $C_1$-$C_4$-Alkyl, Phenyl oder Benzyl bedeutet, $R^2$ einen Rest der Formeln II oder IIa

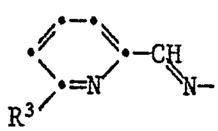

(II),

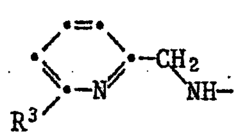

(IIa),

darstellt, worin $R^3$ für H oder $-CH_3$ steht, oder $R^1$ und $R^2$ zusammen ein Rest der Formel

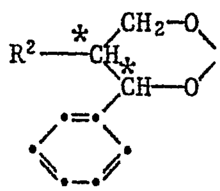

sind, worin $R^2$ die zuvor angegebene Bedeutung hat; und * für überwiegend R- oder überwiegend S-Konfiguration steht.

2. Verbindungen der Formel I gemäss Anspruch 1, worin $R^1$ Methyl oder Benzyl ist.

3. Verfahren zur Herstellung von Verbindungen der Formel I gemäss Anspruch 1, dadurch gekennzeichnet, dass man zur Herstellung der Verbindungen der Formel I, worin $R^2$ ein Rest der Formel II ist, einen Pyridinaldehyd der Formel III

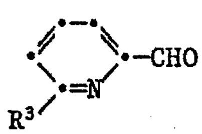

(III)

mit einem Amin der Formel IV

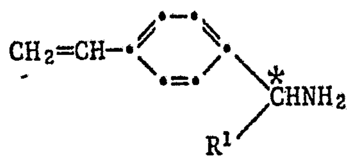

(IV)

oder mit einem Amin der Formel V

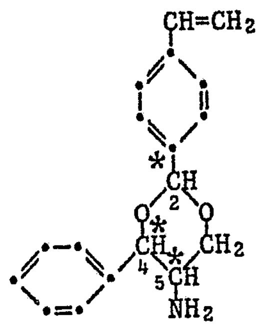

(V)

umsetzt, und zur Herstellung von Verbindungen der Formel I, worin $R^2$ ein Rest der Formel IIa ist, die gebildeten Pyridinaldimine mit $LiAlH_4$ hydriert, wobei $R^1$ und $R^3$ die in Anspruch 1 angegebene Bedeutung haben.

4. Iridiumkomplexe mit Verbindungen der Formel I gemäss Anspruch 1, dadurch gekennzeichnet, dass der Komplex den Formeln VI oder VIa entspricht,

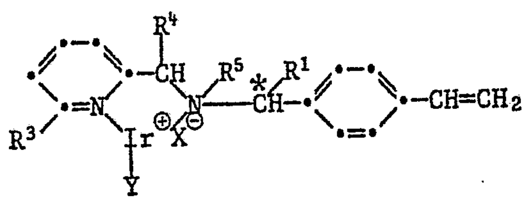

(VI),

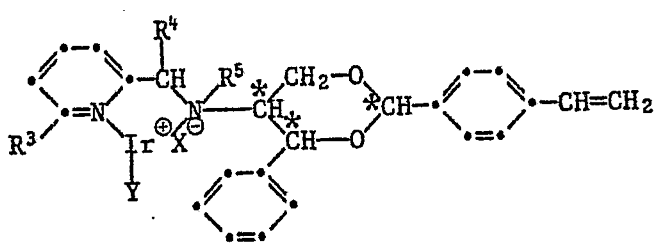

(VIa),

worin * für überwiegend R- oder überwiegend S-Konfiguration steht, $R^1$ und $R^3$ die in Anspruch 1 angegebene Bedeutung haben, $R^4$ und $R^5$ je für H stehen oder zusammen eine Bindung darstellen, $X^\ominus$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten.

5. Iridiumkomplexe gemäss Anspruch 4, worin $R^1$ in Formel VI Methyl oder Benzyl ist.

6. Iridiumkomplexe gemäss Anspruch 4, worin Y in den Formeln VI und VIa ein Dien mit 6 bis 8 C-Atomen darstellt, dessen Diengruppen über 2 C-Atome verbunden sind.

7. Iridiumkomplexe gemäss Anspruch 4, worin Y in den Formeln VI und VIa für Cycloocta-1,5-dien, Norbornadien oder Hexa-1,5-dien stehen.

8. Iridiumkomplexe gemäss Anspruch 4, worin $X^\ominus$ in den Formeln VI und VIa Halogenid, $BF_4^\ominus$, $ClO_4^\ominus$, $CF_3SO_3^\ominus$ oder $PF_6^\ominus$ ist.

9. Iridiumkomplexe gemäss Anspruch 4, worin $X^\ominus$ in den Formeln VI und VIa $Cl^\ominus$, $Br^\ominus$, $J^\ominus$, $BF_4^\ominus$, $ClO_4^\ominus$, $CF_3SO_3^\ominus$ oder $PF_6^\ominus$ ist, und $R^4$ und $R^5$ zusammen für eine Bindung stehen.

10. Iridiumkomplexe gemäss Anspruch 4, worin $X^\ominus$ in den Formeln VI und VIa $BF_4^\ominus$, $ClO_4^\ominus$, $CF_3SO_3^\ominus$ oder $PF_6^\ominus$ ist und $R^4$ und $R^5$ zusammen je für H stehen.

11. Iridiumkomplexe gemäss Anspruch 4 der Formeln

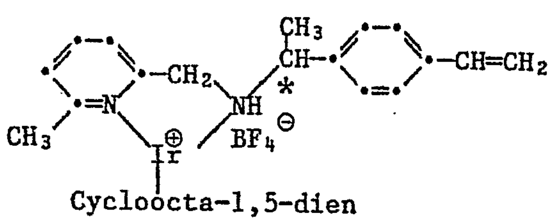 ,

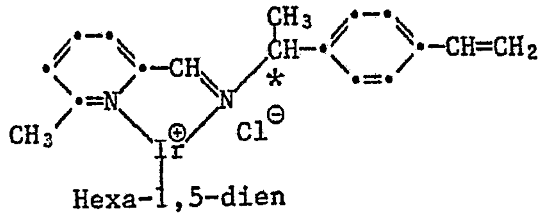
,

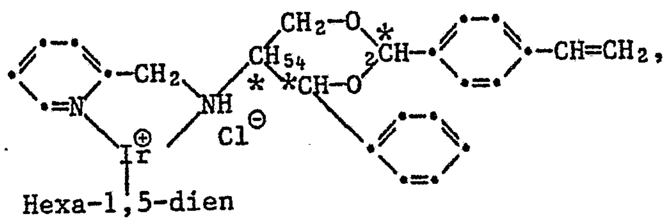

worin * für überwiegend R-Konfiguration bzw. 2R,4S,5S-Konfiguration steht.

12. Homo- und Copolymere mit optisch aktiven Seitengruppen, enthaltend, bezogen auf die Polymeren
a) 0,005 bis 100 Mol-% mindestens eines wiederkehrenden Strukturelementes der Formeln VII

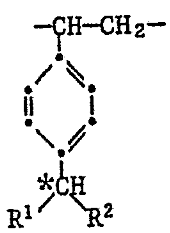
(VII),

worin $R^1$ $C_1$-$C_4$-Alkyl, Phenyl oder Benzyl bedeutet, $R^2$ einen Rest der Formeln II oder IIa

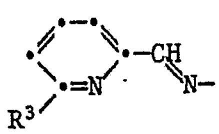
(II),
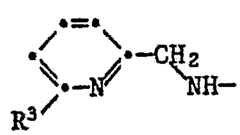
(IIa),

darstellt, worin $R^3$ für H oder $-CH_3$ steht, oder $R^1$ und $R^2$ zusammen ein Rest der Formel

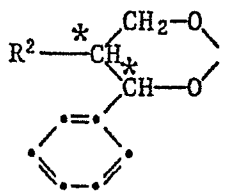

sind, worin $R^2$ die zuvor angegebene Bedeutung hat; und * für überwiegend R- oder überwiegend S-Konfiguration steht, und

b) 99,995 bis 0 Mol-% mindestens eines von einem olefinischen Comonomer abgeleiteten Strukturelementes, das von Komponente a) verschieden ist.

13. Polymere gemäss Anspruch 12, worin $R^1$ in Formel VII für Methyl oder Benzyl steht.

14. Polymere gemäss Anspruch 12, die
a) 0,05 bis 100 Mol-% der Strukturelemente der Formel VII und
b) 99,95 bis 0 Mol-% Strukturelemente eines olefinischen Comonomers enthalten.

15. Polymere gemäss Anspruch 14, die
a) 0,1 bis 10 Mol-% der Strukturelemente der Formel VII und
b) 99,9 bis 90 Mol-% Strukturelemente eines olefinischen Comonomers enthalten.

16. Polymere gemäss Anspruch 12, in denen das Strukturelement der Komponente b) der Formel VIII

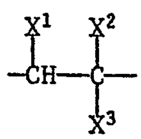 (VIII)

entspricht, worin $X^1$ Wasserstoff, $X^2$ Wasserstoff, Chlor oder Methyl und $X^3$ Wasserstoff, Methyl, Chlor, -CN, $-CONR^7R^8$, worin $R^7$ und $R^8$ unabhängig H oder $C_1$-$C_{18}$-Alkyl sind, Phenyl, Methylphenyl, Methoxyphenyl, Cyclohexyl, -COO-Alkyl mit 1 - 12 C-Atomen im Alkyl, -COO-Phenyl, -COO-Alkyl-OH mit 2 - 6 C-Atomen im Alkyl, -OCO-Alkyl

mit 1 - 4 C-Atomen im Alkyl, -OCO-Phenyl, Alkoxy mit 1 - 20 C-Atomen oder Phenoxy, oder $X^2$ Wasserstoff und $X^1$ und $X^3$ zusammen eine Gruppierung $-CO-NR^6-CO-$ oder je -COO-Alkyl mit 1 - 6 C-Atomen im Alkyl darstellen, wobei $R^6$ geradkettiges oder verzweigtes $C_1$-$C_{18}$-Alkyl, Cyclohexyl oder Phenyl bedeutet, das durch $C_1$-$C_6$-Alkyl, Halogen, Cyano, Nitro und/oder $C_1$-$C_3$-Alkoxy mono- oder disubstituiert sein kann.

17. Polymere gemäss Anspruch 16, worin in Formel VIII $X^1$ für H steht, $X^2$ H oder $-CH_3$ bedeutet und $X^3$ Phenyl, Cyclohexyl, -COO-Alkyl mit 1 - 12 C-Atomen im Alkyl, -COO-Hydroxyalkyl mit 2 bis 4 C-Atomen im Alkyl, oder $-CONR^7R^8$ darstellt, worin $R^7$ und $R^8$ unabhängig für H oder $C_{1-12}$-Alkyl steht.

18. Polymere gemäss Anspruch 16, worin in Formel VIII $X^1$ und $X^2$ für H und $X^3$ für Phenyl, oder $X^1$ für H, $X^2$ für $-CH_3$ und $X^3$ für -COO-Alkyl mit 1 bis 12 C-Atomen im Alkyl stehen.

19. Polymere gemäss Anspruch 12, dadurch gekennzeichnet, dass sie, bezogen auf die vorhandenen Monomeren, bis zu 60 Mol-% Strukturelemente eines zwei- oder mehrfach olefinisch ungesättigten Vernetzungsmittels enthalten.

20. Polymere gemäss Anspruch 19, dadurch gekennzeichnet, dass sie 0,1 bei 10 Mol-% Vernetzungsmittel enthalten.

21. Polymere gemäss Anspruch 19, dadurch gekennzeichnet, dass sie ein Divinylbenzol als Vernetzungsmittel enthalten.

22. Polymere gemäss Anspruch 12, dadurch gekennzeichnet, dass mindestens ein Teil der Strukturelemente der Formel VII mit Iridium (I) komplexiert sind und den Formeln IX oder X entsprechen,

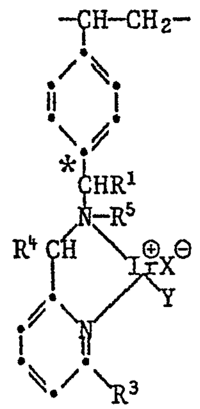 (IX) oder 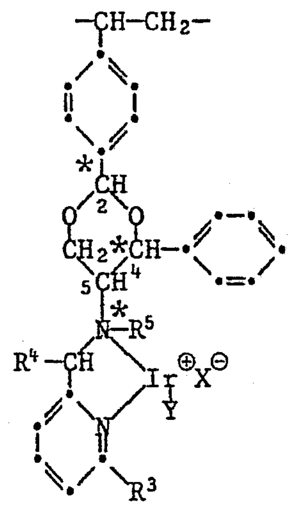 (X),

worin $R^1$ $C_1$-$C_4$-Alkyl, Phenyl oder Benzyl bedeutet, $R^3$ H oder $-CH_3$ sind, $R^4$ und $R^5$ je für H stehen oder zusammen eine Bindung darstellen, $X^{\ominus}$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten.

23. Verfahren zur Herstellung von Polymeren gemäss Anspruch 12, dadurch gekennzeichnet, dass man
a) 0,005 bis 100 Mol-% mindestens eines Monomers der Formel I gemäss Anspruch 1 und
b) 0 bis 99,995 Mol-% mindestens eines olefinischen Comonomers, das von Komponente a) verschieden ist,
polymerisiert.

24. Verfahren zur Herstellung von mit Iridium (I) komplexierten Polymeren gemäss Anspruch 22, dadurch gekennzeichnet, dass man
A) a) 0,005 bis 100 Mol-% mindestens eines Monomers der Formeln VI oder VIa gemäss Anspruch 4 gegebenenfalls zusammen mit einem Monomer der Formel I gemäss Anspruch 1, und
b) 0 bis 99,995 Mol-% mindestens eines olefinischen Comonomers, das von Komponente a) verschieden ist,
polymerisiert, oder

B) dass man in Lösung ein Homo- oder Copolymer gemäss Anspruch 12 mit [(Acetonitril)$_2$(Y)]IrX oder mit Di-[μ-Chlorotetrakis(cyclo-octen)-diiridium (I)] Cl und einem Dien Y umsetzt, wobei X das Anion einer einbasischen anorganischen oder organischen Säure ist.

25. Verwendung von Iridiumkomplexen der Formeln VI und VIa gemäss Anspruch 4 als homogene enantioselektive Katalysatoren für die Transferhydrierung von prochiralen Ketonen mit sekundären Alkoholen.

26. Verwendung von komplexierten Polymeren gemäss Anspruch 22 als homogene oder heterogene enantioselektive Katalysatoren für die Transferhydrierung von prochiralen Ketonen mit sekundären Alkoholen.

27. Reine stereoisomere optisch aktive Amine der Formeln IV oder V

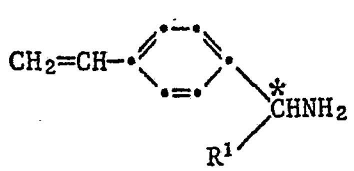

(IV),

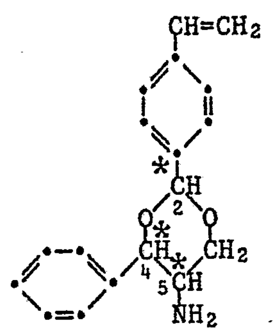

(V)

worin R[1] $C_1$-$C_4$-Alkyl, Phenyl oder Benzyl bedeutet und das chirale *C-Atom in Formel IV entweder R- oder S-Konfiguration aufweist und die chiralen C-Atome in Formel V 2R,4S,5S-Konfiguration aufweisen.

FO 7.3/DA/cw*

**Patentansprüche für den Vertragsstaat: AT**

1. Verfahren zur Herstellung von optisch aktiven Verbindungen der Formel I

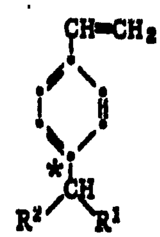

(I),

worin $R^1$ $C_1$-$C_4$-Alkyl, Phenyl oder Benzyl bedeutet, $R^2$ einen Rest der Formeln II oder IIa

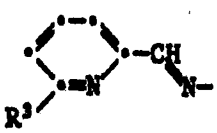

(II),

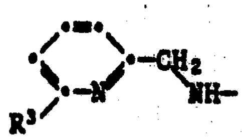

(IIa),

darstellt, worin $R^3$ für H oder $-CH_3$ steht, oder $R^1$ und $R^2$ zusammen ein Rest der Formel

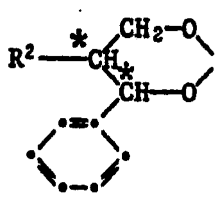

sind, worin $R^2$ die zuvor angegebene Bedeutung hat; und * für überwiegend R- oder überwiegend S-Konfiguration steht, dadurch gekennzeichnet, dass man zur Herstellung der Verbindungen der Formel I, worin $R^2$ ein Rest der Formel II ist, einen Pyridinaldehyd der Formel III

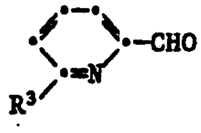

(III)

mit einem Amin der Formel IV

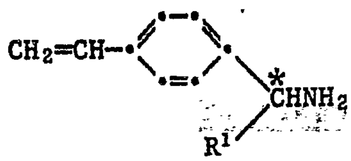

(IV)

oder mit einem Amin der Formel V:

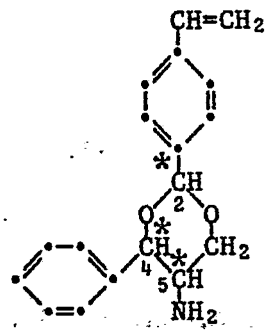

(V)

umsetzt, und zur Herstellung von Verbindungen der Formel I, worin $R^2$ ein Rest der Formel IIa ist, die gebildeten Pyridinaldimine mit $LiAlH_4$ hydriert, wobei $R^1$ und $R^3$ die zuvor angegebenen Bedeutungen haben.

2. Verfahren gemäss Anspruch 1, worin in Formel I $R^1$ Methyl oder Benzyl ist.

3. Verfahren zur Herstellung von Iridiumkomplexen der Formeln VI oder VIa

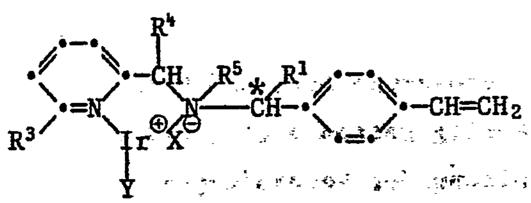

(VI),

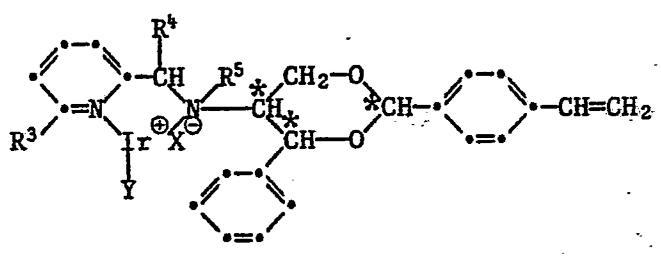

(VIa),

worin * für überwiegend R- oder überwiegend S-Konfiguration steht, $R^1$ und $R^3$ die in Anspruch 1 angegebene Bedeutung haben, $R^4$ und $R^5$ je für H stehen oder zusammen eine Bindung darstellen, $X^\ominus$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten, dadurch gekennzeichnet, dass man [(Acetonitril)(Y)]IrX oder $[IR(Y)Cl]_2$ mit einer Verbindung der Formel I gemäss Anspruch I umsetzt.

4. Verfahren gemäss Anspruch 3, worin $R^1$ in Formel VI Methyl oder Benzyl ist.

5. Verfahren gemäss Anspruch 3, worin Y in den Formeln VI und VIa ein Dien mit 6 bis 8 C-Atomen darstellt, dessen Diengruppen über 2 C-Atome verbunden sind.

6. Verfahren gemäss Anspruch 3, worin Y in den Formeln VI und VIa für Cycloocta-1,5-dien, Norbornadien oder Hexa-1,5-dien stehen.

7. Verfahren gemäss Anspruch 3, worin $X^\ominus$ in den Formeln VI und VIa Halogenid, $BF_4^\ominus$, $ClO_4^\ominus$, $CF_3SO_3^\ominus$ oder $PF_6^\ominus$ ist.

8. Verfahren gemäss Anspruch 3, worin $X^\ominus$ in den Formeln VI und VIa $Cl^\ominus$, $Br^\ominus$, $J^\ominus$, $BF_4^\ominus$, $ClO_4^\ominus$, $CF_3SO_3^\ominus$ oder $PF_6^\ominus$ ist, und $R^4$ und $R^5$ zusammen für eine Bindung stehen.

9. Verfahren gemäss Anspruch 3, worin $X^\ominus$ in den Formeln VI und VIa $BF_4^\ominus$, $ClO_4^\ominus$, $CF_3SO_3^\ominus$ oder $PF_6^\ominus$ ist und $R^4$ und $R^5$ zusammen je für H stehen.

10. Verfahren gemäss Anspruch 3, worin die Komplexe den Formeln

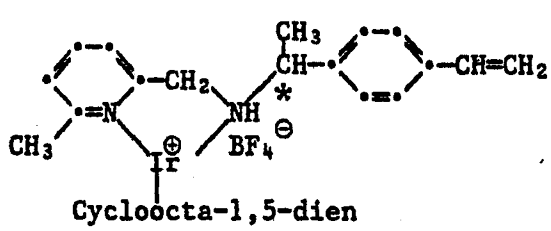

,

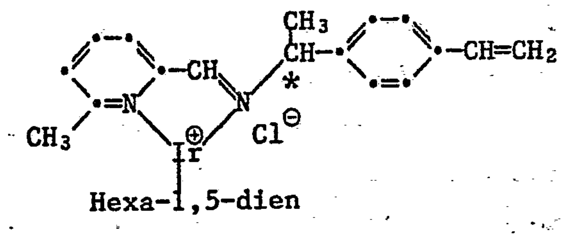
,

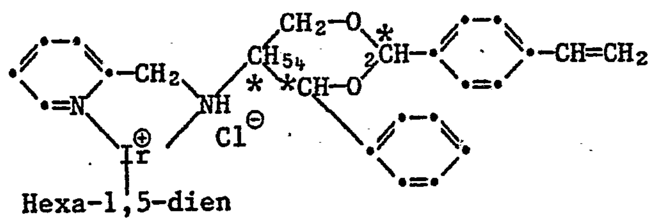

entsprechen, worin * für überwiegend R-Konfiguration bzw. 2R,4S,5S-Konfiguration steht.

11. Verfahren zur Herstellung von Homo- und Copolymeren mit optisch aktiven Seitengruppen, enthaltend, bezogen auf die Polymeren
a) 0,005 bis 100 Mol-% mindestens eines wiederkehrenden Strukturelementes der Formeln VII

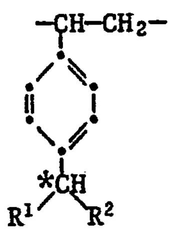
(VII),

worin $R^1$ $C_1$-$C_4$-Alkyl, Phenyl oder Benzyl bedeutet, $R^2$ einen Rest der Formeln II oder IIa

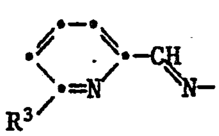
(II),

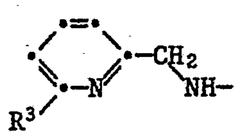
(IIa),

darstellt, worin $R^3$ für H oder $-CH_3$ steht, oder $R^1$ und $R^2$ zusammen ein Rest der Formel

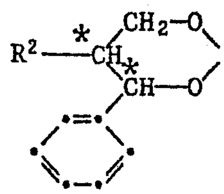

sind, worin $R^2$ die zuvor angegebene Bedeutung hat; und * für überwiegend R- oder überwiegend S-Konfiguration steht, und

b) 99,995 bis 0 Mol-% mindestens eines von einem olefinischen Comonomer abgeleiteten Strukturelementes, das von Komponente a) verschieden ist, dadurch gekennzeichnet, dass man ein Styrolmonomer der Formel I gemäss Anspruch 1 gegebenenfalls zusammen mit einem olefinischen Comonomer polymerisiert.

12. Verfahren gemäss Anspruch 11, worin $R^1$ in Formel VII für Methyl oder Benzyl steht.

13. Verfahren gemäss Anspruch 11, wobei das Polymer
a) 0,05 bis 100 Mol-% der Strukturelemente der Formel VII und
b) 99,95 bis 0 Mol-% Strukturelemente eines olefinischen Comonomers enthalten.

14. Verfahren gemäss Anspruch 13, wobei das Polymer
a) 0,1 bis 10 Mol-% der Strukturelemente der Formel VII und
b) 99,9 bis 90 Mol-% Strukturelemente eines olefinischen Comonomers enthalten.

15. Verfahren gemäss Anspruch 11, wobei im Polymer das Strukturelement der Komponente b) der Formel VIII

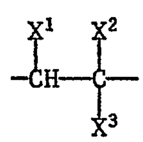

(VIII)

entspricht, worin $X^1$ Wasserstoff, $X^2$ Wasserstoff, Chlor oder Methyl und $X^3$ Wasserstoff, Methyl, Chlor, -CN, $-CONR^7R^8$, worin $R^7$ und $R^8$ unabhängig H oder $C_1$-$C_{18}$-Alkyl sind, Phenyl, Methylphenyl, Methoxy-

phenyl, Cyclohexyl, -COO-Alkyl mit 1 - 12 C-Atomen im Alkyl, -COO-Phenyl, -COO-Alkyl-OH mit 2 - 6 C-Atomen im Alkyl, -OCO-Alkyl mit 1 - 4 C-Atomen im Alkyl, -OCO-Phenyl, Alkoxy mit 1 - 20 C-Atomen oder Phenoxy, oder $X^2$ Wasserstoff und $X^1$ und $X^3$ zusammen eine Gruppierung $-CO-NR^6-CO-$ oder je -COO-Alkyl mit 1 - 6 C-Atomen im Alkyl darstellen, wobei $R^6$ geradkettiges oder verzweigtes $C_1$-$C_{18}$-Alkyl, Cyclohexyl oder Phenyl bedeutet, das durch $C_1$-$C_6$-Alkyl, Halogen, Cyano, Nitro und/oder $C_1$-$C_3$-Alkoxy mono- oder disubstituiert sein kann.

16. Verfahren gemäss Anspruch 15, worin in Formel VIII $X^1$ für H steht, $X^2$ H oder $-CH_3$ bedeutet und $X^3$ Phenyl, Cyclohexyl, -COO-Alkyl mit 1 - 12 C-Atomen im Alkyl, -COO-Hydroxyalkyl mit 2 bis 4 C-Atomen im Alkyl, oder $-CONR^7R^8$ darstellt, worin $R^7$ und $R^8$ unabhängig für H oder $C_{1-12}$-Alkyl steht.

17. Verfahren gemäss Anspruch 15, worin in Formel VIII $X^1$ und $X^2$ für H und $X^3$ für Phenyl, oder $X^1$ für H, $X^2$ für $-CH_3$ und $X^3$ für -COO-Alkyl mit 1 bis 12 C-Atomen im Alkyl stehen.

18. Verfahren gemäss Anspruch 11, dadurch gekennzeichnet, dass das Polymer bezogen auf die vorhandenen Monomeren, bis zu 60 Mol-% Strukturelemente eines zwei- oder mehrfach olefinisch ungesättigten Vernetzungsmittels enthalten.

19. Verfahren gemäss Anspruch 18, dadurch gekennzeichnet, dass das Polymer 0,1 bei 10 Mol-% Vernetzungsmittel enthalten.

20. Verfahren gemäss Anspruch 18, dadurch gekennzeichnet, dass das Polymer ein Divinylbenzol als Vernetzungsmittel enthalten.

21. Verfahren zur Herstellung von mit Iridium (I) komplexierten Polymeren bei denen mindestens ein Teil der Strukturelemente der Formel VII gemäss Anspruch 12 mit Iridium (I) komplexiert sind und den Formeln IX oder X entsprechen,

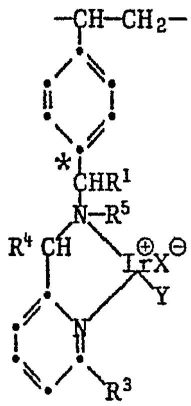 (IX) oder 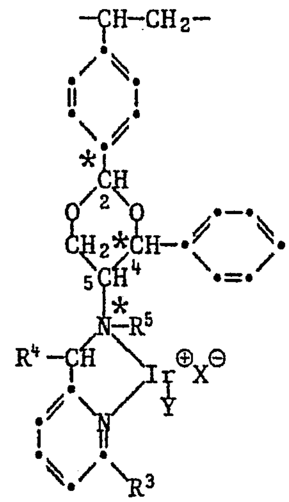 (X),

worin $R^1$ $C_1$-$C_4$-Alkyl, Phenyl oder Benzyl bedeutet, $R^3$ H ode r $-CH_3$ sind, $R^4$ und $R^5$ je für H stehen oder zusammen eine Bindung darstellen, $X^\ominus$ für das Anion einer einbasischen anorganischen oder organischen Säure und Y ein offenkettiges oder cyclisches Dien mit 6 bis 10 C-Atomen, dessen Diengruppen über 1 oder 2 C-Atome verbunden sind, bedeuten, dadurch gekennzeichnet, dass man

A) a) 0,005 bis 100 Mol-% mindestens eines Monomers der Formeln VI oder VIa gemäss Anspruch 4 gegebenenfalls zusammen mit einem Monomer der Formel I gemäss Anspruch 1, und

b) 0 bis 99,995 Mol-% mindestens eines olefinischen Comonomers, das von Komponente a) verschieden ist,

polymerisiert, oder

B) dass man in Lösung ein Homo- oder Copolymer gemäss Anspruch 12 mit $[(Acetonitril)_2(Y)]IrX$ oder mit Di-[µ-Chlorotetrakis(cycloocten)-diiridium (I)] Cl und einem Dien Y umsetzt, wobei X das Anion einer einbasischen anorganischen oder organischen Säure ist.

22. Verwendung von Iridiumkomplexen der Formeln VI und VIa gemäss Anspruch 3 als homogene enantioselektive Katalysatoren für die Transferhydrierung von prochiralen Ketonen mit sekundären Alkoholen.

23. Verwendung von komplexierten Polymeren gemäss Anspruch 21 als homogene oder heterogene enantioselektive Katalysatoren für die Transferhydrierung von prochiralen Ketonen mit sekundären Alkoholen.

FO 7.3/DA/cw*

0251994

Europäisches Patentamt

# EUROPÄISCHER RECHERCHENBERICHT

Nummer der Anmeldung

EP 87810297.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB - A - 1 188 154 (EGYESULT) * Ansprüche 1,16,17 * -- | 1,3 | C 07 D 213/53<br>C 07 D 213/38<br>C 07 D 319/06<br>C 07 D 405/12<br>C 07 C 87/28<br>C 08 F 26/00<br>B 01 J 31/00 |
| A | US - A - 4 383 122 (LAIDLER) * Ansprüche 1,2,6,10; Zusammenfassung * -- | 1,3,4,25 | |
| A | DE - A1 -2 851 039 (BASF) * Seite 2, Zeilen 8-18 * ---- | 1,27 | |
| | | | RECHERCHIERTE SACHGEBIETE (Int. Cl.4) |
| | | | C 07 D 213/00<br>C 07 D 319/00<br>C 07 D 405/00<br>C 07 C 87/00 |

Der vorliegende Recherchenbericht wurde für alle Patentansprüche erstellt.

| Recherchenort | Abschlußdatum der Recherche | Prüfer |
|---|---|---|
| WIEN | 11-08-1987 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument